# EUROPEAN PATENT SPECIFICATION

(11) **EP 3 539 532 B1**
(45) Date of publication and mention of the grant of the patent: **14.09.2022**
(21) Application number: 17869733.0
(22) Date of filing: 14.11.2017
(51) Int. Cl.: A61K 8/891, A61K 8/06, A61K 8/44, A61K 8/60, A61K 8/81, A61K 8/86, A61K 8/894, A61Q 1/00, A61Q 19/00, A61K 8/49, A61K 8/895

(54) **SKIN CARE COMPOSITION**
HAUTPFLEGEZUSAMMENSETZUNG
COMPOSITION POUR LE SOIN DE LA PEAU

(30) Priority: 14.11.2016 JP 2016221654; 01.09.2017 JP 2017168872
(43) Date of publication of application: 18.09.2019
(73) Proprietor: Shiseido Company, Ltd., Tokyo 104-0061 (JP)
(72) Inventor: TAKAHASHI, Shigeo, Yokohama-shi Kanagawa 224-8558 (JP); FURUKAWA, Ryo, Yokohama-shi Kanagawa 224-8558 (JP); DAIDOGUCHI, Noriko, Yokohama-shi Kanagawa 224-8558 (JP); SUZUKI, Kazuaki, Yokohama-shi Kanagawa 224-8558 (JP); YANAI, Motohiro, Yokohama-shi Kanagawa 224-8558 (JP)
(74) Representative: Ter Meer Steinmeister & Partner
(86) International application number: PCT/JP2017/040974
(87) International publication number: WO 2018/088570

(56) References cited:
- EP-A1- 2 796 126
- EP-A1- 3 028 691
- WO-A1-2005/115311
- WO-A1-2013/047196
- WO-A1-2016/052277
- WO-A1-2016/052277
- WO-A2-2009/112492
- JP-A- 2006 213 730
- JP-A- 2011 149 017
- JP-A- 2011 513 463
- JP-A- 2013 082 686
- JP-A- 2014 024 834
- JP-A- 2014 108 934
- JP-A- 2015 030 705
- JP-A- 2015 218 113
- JP-A- 2017 178 806
- US-A1- 2011 117 146

## Description

### RELATED APPLICATION

### FIELD OF THE INVENTION

The present disclosure relates to an external oil-in-water emulsion composition for skin.

### BACKGROUND OF THE INVENTION

Compositions comprising silicone elastomers are known (for example, refer to Patent Literatures 1 and 2). Patent Literature 1 discloses an oil-in-water emulsified external composition for skin comprising: (a) a water-soluble thickener having a hydrophobic group; (b) a nonionic surfactant having an HLB of 14 or more; (c) a polyether-modified silicone having an HLB of 3 to 5; and (d) a non-emulsifying type crosslinked silicone.

Patent Literature 2 discloses a cosmetic comprising 15.0% by weight of tris(trimethylsiloxy)isobutyl silane, 1.0% by weight of a nonionic surfactant which is polyglycerin-modified organopolysiloxane, 1.5% by weight of a crosslinked organopolysiloxane having a polyglycerin moiety in the crosslinked structure thereof, and water (refer to Example 23 in paragraph [0153]).

### CITATION LIST

### PATENT LITERATURE

PATENT LITERATURE 1: JP Patent Kokai Publication No. JP2013-82686A
PATENT LITERATURE 2: JP Patent Kokai Publication No. JP2006-213730A

### DISCLOSURE OF THE INVENTION

### TECHNICAL PROBLEM

When silicone elastomers are added to compositions of cosmetics and the like, smooth and rich feeling in use can be provided to users. However, silicone elastomers aggregate easily in the composition. For example, in the oil-in-water emulsified external composition for skin described in Patent Literature 1, dispersibility of the silicone elastomer is deteriorated when the viscosity becomes less than 10,000 mPa·s. Accordingly, in a composition that comprises silicone elastomers as described in Patent Literature 1, a thickener is added to make the composition into a high viscous cream form so that movement of silicone elastomers in the composition is suppressed and thus aggregation of silicone elastomers is suppressed. However, in the high viscous composition in a cream form as such, it is difficult to achieve light and dewy feeling in use like in compositions in a gel-form or a milk-form.

The crosslinked organopolysiloxane having a polyglycerin moiety in the crosslinked structure described in Patent Literature 2 can be expressed as an emulsifying type silicone elastomer. If the emulsifying type silicone elastomer is included in an external composition for skin, stickiness occurs when the composition is applied to skin and the user cannot obtain smooth feeling in use.

Thus, there is a demand for a composition of which aggregation of silicone elastomers is suppressed even if the composition is low-viscous.

Furthermore, there is a demand for an external composition for skin that the user can obtain dewy, non-sticky and smooth feeling in use upon application to skin.

### SOLUTION TO THE PROBLEM

According to a first aspect of the present disclosure, an external oil-in-water emulsion composition for skin comprises 0.1% by mass to 8% by mass of a non-emulsifying silicone elastomer, a polyether-modified silicone surfactant and a hydrophilic nonionic surfactant. The hydrophilic nonionic surfactant comprises a glycerin-derived surfactant. The polyether-modified silicone surfactant is 0.3 part by mass or more with respect to 1 part by mass of the silicone elastomer. The hydrophilic nonionic surfactant is 0.2 part by mass or more with respect to 1 part by mass of the silicone elastomer. In cases where an amphoteric surfactant is included, the amphoteric surfactant is 1.5 parts by mass or less with respect to 1 part by mass of the glycerin-derived surfactant. The viscosity of the composition is 9,500 mPa·s or less.

### EFFECT OF THE INVENTION

According to the present disclosure, aggregation of the silicone elastomer can be suppressed and the silicone elastomer can be uniformly dispersed even if the viscosity of the composition is low. Accordingly, smooth and rich feeling in use provided by the silicone elastomer and dewy feeling in use can be achieved at the same time.

According to the present disclosure, stickiness that the user feels upon application can be suppressed while the above-mentioned feeling in use is achieved.

### MODES FOR CARRYING OUT THE INVENTION

Preferred modes of the above-mentioned aspects will be described in the following.

According to a preferred mode of the first aspect, the external composition for skin comprises an amphoteric surfactant. The amphoteric surfactant is 0.15 part by mass to 1.5 parts by mass with respect to 1 part by mass of a glycerin-derived surfactant.

According to a preferred mode of the first aspect the amphoteric surfactant comprises lauryl dimethylamidoacetic acid betaine.

According to a preferred mode of the first aspect the polyether-modified silicone surfactant has a structure in which a plurality of hydrophilic groups having a polyether group is grafted from a main chain.

According to a preferred mode of the first aspect the main chain comprises a silicone chain.

According to a preferred mode of the first aspect the polyether-modified silicone surfactant comprises a polyoxyethylene methylpolysiloxane copolymer.

According to a preferred mode of the first aspect the glycerin-derived surfactant comprises a polyoxyethylene glycerin fatty acid ester.

According to a preferred mode of the first aspect the hydrophilic nonionic surfactant further comprises a sorbitan-derived surfactant. The sorbitan-derived surfactant is 0.5 part by mass to 5 parts by mass with respect to 1 part by mass of the glycerin-derived surfactant.

According to a preferred mode of the first aspect the external composition for skin further comprises a silicone oil.

According to a preferred mode of the first aspect the average particle size of the silicone elastomer is 100 µm or less.

According to a preferred mode of the first aspect the external composition for skin further comprises a polymer and/or a copolymer (including a crosslinked polymer) having 2-acrylamido-2-methylpropane sulfonic acid or a salt thereof as a constituent unit.

According to a preferred mode of the first aspect the content by percentage of the silicone elastomer is 5% by mass or less with respect to the mass of the composition.

According to a preferred mode of the first aspect the polyether-modified silicone surfactant is 0.5 part by mass or more with respect to 1 part by mass of the silicone elastomer.

According to a preferred mode of the first aspect the silicone elastomer is a crosslinked organopolysiloxane that does not have a polyoxyalkylene group.

According to a preferred mode of the first aspect the silicone elastomer is in a form of an indefinite shaped particle.

According to a preferred mode of the first aspect the silicone elastomer is in a gel-state.

The composition of the present disclosure is applicable to cosmetics, for example. The composition of the present disclosure may is an oil-in-water type.

In the following description, POE is an abbreviation of polyoxyethylene, POP is an abbreviation of polyoxypropylene, and the number in parentheses following POE or POP indicates the average number of moles of POE groups or POP groups added in the respective compound.

In the present disclosure, "practical amount" refers to an amount that can provide any function/effect achieved by the addition of the compound in question.

Aggregation of the silicone elastomer as used herein refers to a state where the silicone elastomer cannot be uniformly dispersed in the composition due to aggregation caused by the movement of the silicone elastomer in the compostion. For example, when the particle size of the silicone elastomer particle coalesces and the particle size thereof is enlarged, dispersibility will deteriorate and smoothness in appearance will not be achieved.

Stability of emulsification as used herein refers to a state where an emulsified particle that is present separately from the silicone elastomer is stably present in the composition and no separation and the like occur. On the other hand, instability of emulsification refers to a state where emulsification becomes unstable by coalescence of emulsified particles by ion repulsion and thus separation and the like occur.

The external compositions for skin according to the present disclosure comprise: (A) a non-emulsifying silicone elastomer; (B) a polyether-modified silicone surfactant; and (C) a hydrophilic surfactant.

### [(A) Non-emulsifying silicone elastomer]

The non-emulsifying type silicone elastomer as used herein refers to a silicone elastomer of which the crosslinking part or the main chain thereof is not mofidied by a polyoxyalkelene group. In particular, it is preferred that the non-emulsifying type silicone elastomer consists of hydrophobic moieties (does not have a hydrophilic moiety). Addition of the emulsifying type silicone elastomer having a polyoxyalkelene group causes stickiness like surfactants and smoothness upon application to skin will be lost. On the other hand, when the non-emulsifying type silicone elastomer is used, stickiness can be suppressed and softness upon application to skin (smooth feeling) can be achieved.

The silicone elastomer applicable to the composition of the present disclosure is not particulary limited as long as it is applicable to skin. Examples of silicone elastomers may include dimethicone crosspolymer, dimethicone/vinyl dimethicone crosspolymer, dimethicone/phenyl vinyl dimethicone crosspolymer, vinyl dimethicone/lauryl dimethicone crosspolymer, lauryl polydimethyl siloxyethyl dimethicone/bis-vinyl dimethicone crosspolymer, alkyl (C30-45) cetearyl dimethicone crosspolymer, cetearyl dimethicone crosspolymer and the like. Dimethicone/vinyl dimethicone crosspolymer also referred to as Polysilicone-11 is preferable among the foregoing examples from the viewpoint of non-aggregation property.

Commercial products, for example, can be used as the silicone elastomer. The commercial product may be a mixture of a silicone elastomer and an oily component. The oily component included in such commercial product is not particularly limited as long as it is applicable to skin. Examples of the commercial product comprising a silicone elastomer may include Gransil DMG-3 (Grant Industries, Inc.) that comprises 12% by mass of Polysilicone-11 as the silicone elastomer and 88% by mass of dimethicone as the oily component, and other products such as KSG-16 (Shin-Etsu Chemical Co., Ltd.), Dow Corning^{®} 9041 Silicone Elastomer Blend (Dow Corning Toray Co., Ltd.) and the like.

The content by percentage of the non-emulsifying silicone elastomer in the composition of the present disclosure is preferably 0.1% by mass or more, more preferably 0.3% by mass or more, more preferably 0.5% by mass or more, and further more preferably 0.7% by mass or more with respect to the mass of the composition. If the content by percentage of the silicone elastomer is less than 0.1% by mass, smooth and rich feeling in use may not be achieved. The content by percentage of the silicone elastomer is preferably 8% by mass or less, preferably 5% by mass or less, more preferably 3% by mass or less, more preferably 2% by mass or less, more preferably 1.5% by mass or less, and further more preferably 1% by mass or less with respect to the mass of the composition.
If the content by percentage of the silicone elastomer exceeds 8% by mass, it becomes difficult to disperse the silicone elastomer stably.

The content by percentage of the non-emulsifying silicone elastomer in the composition of the present disclosure may also be 4% by mass or less or 3% by mass or less with respect to the mass of the composition.

It is preferred that the silicone elastomer is present in a granular form (as a particle) in the composition of the present disclosure. The average particle size of the silicone elastomer particle is preferably 150 µm or less, and more preferably 100 µm or less. If the average particle size is larger than 150 µm, dispersibility of the silicone elastomer will deteriorate and, at the same time, uniformity will be impaired.

It is preferred that the silicone elastomer particle has an indefinite shape in the composition of the present disclosure. The silicone elastomer is preferably in a form of a gel. For example, the silicone elastomer particle can be obtained by finely pulverizing a gel-state lump of the silicone elastomer. Feeling in use with good compatibility to skin can be achieved by making the silicone elastomer in an indefinite shape.

### [(B) Polyether-modified silicone surfactant]

As an example of the polyether-modified silicone surfactant, a polyether-modified silicone in which a polyoxyalkylene group such as polyoxyethylene (POE), polyoxypropylene (POP) and the like is introduced to a silicone skeleton can be used. In particular, it is preferred that the silicone surfactant has a silicone chain (siloxane chain) as a main chain and a hydrophilic group having a polyether group as a side chain. It is preferred that the silicone surfactant has a pendant-shaped (comb-shaped) structure in which a plurality of polyoxyalkelene groups (polyether chain) is introduced to the silicone chain as the side chain. If the silicone surfactant has the pendant-shaped structure, dispersion stability of the silicone elastomer can be enhanced.

For example, a polyoxyethylene methylpolysiloxane copolymer, a cetyl dimethicone copolyol and the like can be used as the silicone surfactant. Examples of commercial products of the polyoxyethylene methylpolysiloxane copolymer may include PEG-10 Dimethicone (Silicone KF-6017P, Shin-Etsu Chemical Co., Ltd.), Cetyl PEG/PPG10-1 Dimethicone (ABIL^{®} EM90, Evonik Industries AG) and the like.

The HLB (Hydrophile-Lipohile Balance) of the silicone surfactant calculated by the Griffin method is preferably 5 or less.

The content by percentage of the non-emulsifying surfactant in the composition of the present disclosure is preferably 0.3% by mass or more, more preferably 0.5% by mass or more, more preferably 0.8% by mass or more, and further more preferably 1% by mass or more with respect to the mass of the composition. If the content by percentage of the silicone surfactant is less than 0.3% by mass, dispersibility of the silicone elastomer will deteriorate. The content by percentage of the silicone surfactant in the composition of the present disclosure may be 5% by mass or less, 4% by mass or less, or 3% by mass or less with respect to the mass of the composition. The content by percentage of the silicone surfactant is preferably 2.5% by mass or less, more preferably 2% by mass or less, and further more preferably 1.5% by mass or less with respect to the mass of the composition. If the content by percentage of the silicone surfactant exceeds 5% by mass, emulsification stability will deteriorate.

With respect to the mass ratio of (A) the non-emulsifying silicone elastomer and (B) the polyether-modified silicone surfactant in the composition of the present disclosure, the mass of (A) is preferably 0.4 or more, more preferably 0.6 or more, and further more preferably 0.8 or more when the mass of (B) is 1. The mass of (A) can be 0.2 or more with respect to the mass of (B). With respect to the mass ratio of (A) the non-emulsifying silicone elastomer and (B) the polyether-modified silicone surfactant, the mass of (A) is preferably 3 or less, more preferably 2 or less, and further more preferably 1.5 or less when the mass of (B) is 1. If the mass ratio is within this range, aggregation of the non-emulsifying silicone can be suppressed.

With respect to the mass ratio of (A) the non-emulsifying silicone elastomer and (B) the polyether-modified silicone surfactant in the composition of the present disclosure, the mass of (B) is preferably 0.3 or more, more preferably 0.5 or more, and further more preferably 0.8 or more when the mass of (A) is 1. If the mass of (B) is less than 0.3, stability of the component (A) will deteriorate. With respect to the mass ratio of (A) the silicone elastomer and (B) the silicone surfactant, the mass of (B) is preferably 5 or less, more preferably 4 or less, and further more preferably 2 or less when the mass of (A) is 1. If the mass of the component (B) exceeds 5, stickiness will occur.

### [(C) Hydrophilic surfactant]

A nonionic surfactant and/or an amphoteric surfactant can be used as the hydrophilic surfactant.

With respect to the mass ratio of (A) the non-emulsifying silicone elastomer and (C) the hydrophilic surfactant in the composition of the present disclosure, the mass of (A) is preferably 0.2 or more, more preferably 0.3 or more, and further more preferably 0.5 or more when the mass of (C) is 1. With respect to the mass ratio of (A) the silicone elastomer and (C) the hydrophilic surfactant, the mass of (A) is preferably 3 or less, more preferably 2 or less, and further more preferably 1.5 or less when the mass of (C) is 1. If the mass ratio is within this range, aggregation of the silicone elastomer can be suppressed.

### [(C1) Nonionic surfactant]

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

The hydrophilic nonionic surfactant can be used in combination with a plurality of surfactants. For example, the nonionic surfactant can comprise at least one of a sorbitan-derived surfactant and a glycerin-derived surfactant.

For example, polyoxyethylene sorbitan fatty acid ester and the like can be used as the sorbitan-derived surfactant. Examples of commercial products of polyoxyethylene sorbitan fatty acid ester may include POE (20) Sorbitan Cocoate (NIKKOL^{®} TL-10, Nikko Chemicals

Co., Ltd.) and the like.

Other examples of the sorbitan-derived surfactant may include: sorbitan laurate (NIKKOL^{®} SL-10 and LM, Nikko Chemicals Co., Ltd.); (EMASOL^{®} 110, Kao Corporation), sorbitan palmitate (NIKKOL^{®} SP-10, Nikko Chemicals Co., Ltd.), sorbitan oleate (NIKKOL^{®} SO-10V, Nikko Chemicals Co., Ltd.), sorbitan stearate (NIKKOL^{®} SS-10, Nikko Chemicals Co., Ltd.), sorbitan isostearate (NIKKOL^{®} SI-10PKV Nikko Chemicals Co., Ltd.), sorbitan sesquioleate (NIKKOL^{®} SO-15, Nikko Chemicals Co., Ltd.); (RHEODOL^{®} AO-15, Kao Corporation); (NOFABLE^{®} SO-852S, NOF CORPORATION), sorbitan trioleate ((RHEODOL^{®} SP-O-30, Kao Corporation); (NOFABLE^{®} SO-853S, NOF CORPORATION)), sorbitan tristearate (NIKKOL^{®} SS-30V, Nikko Chemicals Co., Ltd.), sorbitan sesquiisostearate (ESTEMOL^{®} 182V The Nisshin OilliO Group, Ltd.), Polysorbate 60 (NIKKOL^{®} TS-10V, Nikko Chemicals Co., Ltd.), Polysorbate 80 (RHEODOL^{®} TWO-120, Kao Corporation); (NIKKOL^{®} TO-10V, Nikko Chemicals Co., Ltd.) and the like.

For example, polyoxyethylene glycerin fatty acid ester (polyoxyethylene fatty acid glyceryl), self-emulsifying type glycerin fatty acid ester, alkyl glyceryl ether and the like can be used as the glycerin-derived surfactant.

For example, polyoxyethylene glyceryl isostearate and the like can be used as the glycerin-derived surfactant. Examples of commercial products of polyoxyethylene glyceryl isostearate may include PEG-60 glyceryl isostearate (EMALEX^{®} GWIS160N, Nihon Emulsion Co., Ltd.) and the like.

Other examples of the glycerin-derived surfactant may include: PEG-5 glyceryl stearate (EMALEX^{®} GWIS GM-5, Nihon Emulsion Co., Ltd.), PEG-8 glyceryl isostearate (EMALEX^{®} GWIS-108, Nihon Emulsion Co., Ltd.), PEG-20 glyceryl isostearate (EMALEX^{®} GWIS-120, Nihon Emulsion Co., Ltd.), PEG-60 glyceryl isostearate (EMALEX^{®} GWIS-160N, Nihon Emulsion Co., Ltd.), PEG-90 glyceryl isostearate (EMALEX^{®} GWIS-190, Nihon Emulsion Co., Ltd.), PEG-5 glyceryl triisostearate (EMALEX^{®} GWIS-305, Nihon Emulsion Co., Ltd.), PEG-20 glyceryl triisostearate (EMALEX^{®} GWIS-320EX, Nihon Emulsion Co., Ltd.), PEG-20 glyceryl triisostearate (EMALEX^{®} GWIS-320, Nihon Emulsion Co., Ltd.), PEG-20 hydrogenated castor oil isostearate (EMALEX^{®} RWIS-120, Nihon Emulsion Co., Ltd.), PEG-5 glyceryl triisostearate (EMALEX^{®} RWIS-305, Nihon Emulsion Co., Ltd.), glyceryl stearate (SE) (SUNSOFT^{®} 30 , Taiyo Kagaku Co., Ltd.), glyceryl stearate ((SUNSOFT^{®} 8004, Taiyo Kagaku Co., Ltd.); (NIKKOL^{®} MGS-EEXV, Nikko Chemicals Co., Ltd.)), glyceryl oleate (SUNSOFT^{®} 301V and O-30S, Taiyo Kagaku Co., Ltd.), (RHEODOL^{®} MO-60, Kao Corporation), Cotton oil fatty acid monoglyceride (SUNSOFT^{®} 8080, Taiyo Kagaku Co., Ltd.) and the like.

The HLB of the nonionic surfactant calculated by the Davies method is preferably 10 or more, more preferably 12 or more, and further more preferably 14 or more. If the HLB is less than 10, effect on suppressing aggregation of the silicone elastomer will deteriorate.

The content by percentage of the nonionic surfactant in the composition of the present disclosure is preferably 0.1% by mass or more, more preferably 0.3% by mass of more, more preferably 0.8% by mass or more, and further more preferably 1% by mass or more with respect to the mass of the composition. If the content by percentage of the nonionic surfactant is less than 0.1% by mass, dispersion stability of the silicone elastomer will deteriorate. The content by percentage of the nonionic surfactant may be, for example, 3% by mass or less, 2.5% by mass or less and/or 1.5% by mass or less with respect to the mass of the composition. In cases where the composition comprises a plurality of kinds of nonionic surfactants, the content by percentage is calculated with the total mass of the nonionic surfactant.

Cases where the nonionic surfactant comprises the sorbitan-derived surfactant and the glycerin-derived surfactant are considered. With respect to the mass ratio of the sorbitan-derived surfactant and the glycerin-derived surfactant, the mass of the sorbitan-derived surfactant is preferably 0.5 or more, more preferably 1 or more, more preferably 1.3 or more, and further more preferably 2 or more when the mass of the glycerin-derived surfactant is 1. With respect to the mass ratio of the sorbitan-derived surfactant and the glycerin-derived surfactant, the mass of the sorbitan-derived surfactant is preferably 4 or less, and more preferably 3.5 or less when the mass of the glycerin-derived surfactant is 1. The mass of the sorbitan-derived surfactant may be 5 or less with respect to 1 of the glycerin-derived surfactant. If the mass ratio of the sorbitan-derived surfactant and the glycerin-derived surfactant is within the above-mentioned range, aggregation of the silicone elastomer can be suppressed.

According to the invention as presently claimed, the nonionic surfactant (C1) comprises a glycerin-derived surfactant. It is more preferable to use the glycerin-derived surfactant and the sorbitan-derived surfactant in combination. The glycerin-derived surfactant has a higher effect on suppressing aggregation of (A) the silicone elastomer compared to the sorbitan-derived surfactant. When the glycerin-derived surfactant and the sorbitan-derived surfactant are combined, effect on suppressing aggregation of (A) the silicone elastomer can be further enhanced.

With respect to the mass ratio of (A) the non-emulsifying elastomer and (C1) the nonionic surfactant in the composition of the present disclosure, the mass of (A) is preferably 0.2 or more, more preferably 0.3 or more, and more preferably 0.5 or more when the mass of (C1) is 1. With respect to the mass ratio of (A) the silicone elastomer and (C1) the nonionic surfactant, the mass of (A) is preferably 3 or less, more preferably 2 or less, and further more preferably 1.5 or less when the mass of (C1) is 1. If the mass ratio is within this range, aggregation of the silicone elastomer can be suppressed.

It is preferred to set the content by percentage of (C1) the nonionic surfactant in the composition of the present disclosure in accordance with each embodiment to be described later. For example, it is preferred to determine the range of the content by percentage of (C1) the nonionic surfactant by associating it with the content by percentage of (C2) the amphoteric surfactant. (C1) the nonionic surfactant may not be added according to the content by percentage of (C2) the amphoteric surfactant.

In the present disclosure, "(C1) the nonionic surfactant" and "(B) the silicone surfactant" are different surfactants, and the above-mentioned "(B) the silicone surfactant" is not included in "(C1) the nonionic surfactant" as used herein.

### [(C2) Amphoteric surfactant]

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of commercial products of the amphoteric surfactant may include lauryl dimethylaminoacetic acid betaine (NISSANANON^{®} BL-SF, NOF CORPORATION).

It is preferred to set the content by percentage of the amphoteric surfactant in the composition of the present disclosure in accordance with each embodiment to be described later. For example, it is preferred to determine the range of the content by percentage of the amphoteric surfactant by associating it with the content by percentage of the nonionic surfactant, particularly the glycerin-derived nonionic surfactant. (C2) the amphoteric surfactant may not be added according to the content by percentage of (C1) the nonionic surfactant.

### [(D) Silicone oil]

The composition of the present disclosure can further comprise (D) a silicone oil in addition to the above-mentioned components.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

The content by percentage of the silicone oil in the composition of the present disclosure is preferably 7% by mass or more, more preferably 8% by mass or more, and further more preferably 9% by mass or more with respect to the mass of the composition. If the content by percentage of the silicone oil is 7% by mass or more, dispersibility of the silicone elastomer can be enhanced. The content by percentage of the silicone oil is preferably 16% by mass or less, more preferably 15% by mass or less, and further more preferably 14% by mass or less with respect to the mass of the composition. If the content by percentage of the silicone oil exceeds 16% by mass, emulsification stability will deteriorate.

### [(E) Thickener]

The composition of the present disclosure can further comprise (E) a thickener in addition to the above-mentioned components.

For example, taurate-based synthetic polymer and/or acrylate-based synthetic polymer can be used as the thickener.

For example, as the taurate-based synthetic polymer type thickener, a polymer and/or a copolymer (including a crosspolymer) having 2-acrylamido-2-propanesulphonic acid (acryloyldimethyltaurine) or a salt thereof (AMPS structure) as a constituent unit may be used. As such thickener, for example, at least one selected from Ammonium Acryloyldimethyltaurate/Beheneth-25 Methacrylate Crosspolymer (Aristoflex^{®} HMB, Clariant (Japan) K.K.), Ammonium Acryloyldimethyltaurate/VP Copolymer (Aristoflex^{®} AVC, Clariant (Japan) K.K.), Ammonium Acryloyldimethyltaurate/Carboxyethyl Acrylate Crosspolymer (Aristoflex^{®} TAC, Clariant (Japan) K.K.), Polyacrylate Crosspolymer-11 (Aristoflex^{®} Velvet, Clariant (Japan) K.K.), Dimethylacrylamide/Sodium Acryloyldimethyltaurate Crosspolymer (SU-GEL, TOHO Chemical Co., Ltd.), Hydroxyethyl Acrylate/Sodium Acryloyldimethyl Taurate Copolymer (SEPINOV EMT10 PINOV, SEPPIC Inc.), Sodium Acrylate/Acryloyldimethyltaurate/Dimethylacrylamide Crosspolymer (SEPINOV P88, SEPPIC Inc.), Sodium Acrylate/Sodium Acryloyldimethyl Taurate Copolymer (SIMUGEL, SEPPIC Inc.), Sodium Acryloyldimethyltaurate/Methacrylamidolauric Acid Copolymer (AMO-51, DAITO KASEI KOGYO CO., LTD.) and Aacrylamide/Sodium Acryloyldimethyltaurate/Acrylic Acid Copolymer (ACUDYNE SCP, Dow Chemical Company) can be used.

For example, as the acrylate-based synthetic polymer type thickener, Acrylates/Steareth-20 Methacrylate Copolymer (ACULYN^{®} 22, Dow Chemical Company) and Aacrylates/C10-30 Alkyl Acrylate Crosspolymer (PEMULEN^{®} TR-2) can be used.

The content by percentage of the thickener in the composition of the present disclosure is preferably 0.2% by mass or more, 0.5% by mass or more, and further more preferably 0.8% by mass or more with respect to the mass of the composition. If the content by percentage of the thickener is less than 0.2% by mass, emulsification stability will deteriorate and dispersibility of the silocone elastomer will deteriorate, too. The content by percentage of the thickener is preferably 2% by mass or less, and more preferably 1.5% by mass or less with respect to the mass of the composition. If the content by percentage of the thickener exceeds 2% by mass, feeling in use that is desired for the composition of the present disclosure willbe impaired.

Other thickeners can be added within the range of not inhibiting the effect of the present disclosure. Examples of other thickeners may include gum arabic, carrageenan, karaya gum, tragacanth gum, carob gum, quince seed (marmelo), casein, dextrin, gelatin, sodium pectate, sodium alginate, methylcellulose, ethyl cellulose, carboxymethyl cellulose (CMC), hydroxyethyl cellulose, hydroxypropyl cellulose, polyvinyl alcohol (PVA), polyvinyl methyl ether (PVM), PVP (polyvinyl pyrrolidone), sodium polyacrylate, carboxyvinyl polymer, locustbean gum, guar gum, tamarind gum, dialkyldimethylammonium sulfate cellulose, xanthan gum, magnesium aluminum silicate, bentonite, hectorite, magnesium aluminum silicate (veegum), laponite, silicic anhydride and the like.

### [(F) Others]

The composition of the present disclosure may include, as appropriate and as necessary, other components within the range of not inhibiting the effect of the present disclosure such as aqueous solvents, anionic surfactants, cationic surfactants, lipophilic nonionic surfactants, powder bodies, moisturizers, water-soluble polymers, thickeners, oily components, film-forming agents, ultraviolet absorbers, sequestrants, amino acids, organic amines, polymeric emulsions, pH adjusters, skin nutrients, vitamins, antioxidants, antioxidant aids, perfumes and the like.

Examples of aqueous solvents may include water, alcohols, or mixtures thereof.

Water used in cosmetics, quasi-drugs and the like may be used. For example, purified water, ion-exchanged water, tap water and the like may be used. Depending on the purpose, the aqueous phase may further include a water-soluble alcohol.

Examples of water-soluble alcohols may include at least one type selected from lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides and derivatives of the above.

Examples of the lower alcohol may include ethanol, propanol, isopropanol, isobutyl alcohol, t-butyl alcohol and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitose, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heplose); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyoseverbascoses and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid and the like.

Examples of other polyols may include at least one selected from polyoxyethylene methyl glucoside (Glucum E-10), polyoxypropylene methyl glucoside (Glucum P-10) and the like.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcosinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, and POE-stearylether phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); distearyldimethyl ammonium chloride; dialkyldimethyl ammonium salt; poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride and the like.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether and the like.

The term "powder body" as used herein means the same as "powder". The powder body is not particularly limited as long as it is generally applicable for cosmetics and the like. Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc.) and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,0000, etc); acrylic polymer (such as sodium polyacrylate, polyethylacrylate, polyacrylamide, etc); polyethyleneimine; cationic polymer and the like.

Examples of the oily component that may be used may include liquid oils, solid fats, waxes, hydrocarbons, higher fatty acids, higher alcohols, synthetic ester oils and the like.

Examples of the liquid fat that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether and the like.

Examples of the hydrocarbon oils that may be used may include lsiquid paraffin, ozocerite, squalane, pristane, paraffin, ceresin. squalene, vaseline, microcrystalline wax and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid(EPA), docosahexaenoic acid(DHA) and the like.

Examples of the higher alcohols may include a linear alcohol (such as lauryl alcohol, cetyl alcohol, stearyl alcohol, behenyl alcohol, myristyl alcohol, oleyl alcohol, cetostearyl alcohol, and the like); a branched alcohol (such as monostearyl glycerin ether (batyl alcohol), 2-decyltetradecanol, lanolin alcohol, cholesterol, phytosterol, hexyl dodecanol, isostearyl alcohol, octyldodecanol, and the like) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate and the like.

Examples of the film-forming agents may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc.) and the like.

Examples of the ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc); benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); 3-(4'-methylbenzylidene)-d,l-camphor and 3-benzylidene-d,l-camphor; 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; dimorpholinopyridazinone; 2-ethylhexyl 2-cyano-3,3-diphenylacrylate; 2,4-bis-{[4-(2-ethylhexyloxy)-2-hydroxy]-phenyl}-6-(4-methoxyphenyl)-(1,3,5)-triazine and the like.

Examples of the sequestrants may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate and the like.

Examples of the amino acids may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate and the like.

Examples of the organic amines may include monoethanolamine, diethanolamine, triethanolamine, morpholine, triisopropanolamine, 2-amino-2-methyl-1,3-propanediol, 2-amino-2-methyl-1-propanol and the like.

Examples of the polymer emulsions may include acrylic resin emulsion, ethyl polyacrylate emulsion, solution of acrylic resin, polyacrylalkylester emulsion, polyvinyl acetate resin emulsion, natural rubber latex and the like.

Examples of the pH adjusters may include buffer such as lactic acid-sodium lactate, citric acid-sodium citrate, succinic acid-sodium succinate and the like.

Examples of the vitamins may include vitamine A, B1, B2, B6, C, E and derivatives thereof, pantothenic acid and derivatives thereof, biotin and the like.

Examples of the anti-oxidants may include tocopherols, dibutyl hydroxy toluene, butyl hydroxy anisole, and gallic acid esters and the like.

Examples of the anti-oxidant aids may include phosphoric acid, citric acid, ascorbic acid, maleic acid, malonic acid, succinic acid, fumaric acid, cephalin, hexamethaphosphate, phytic acid, ethylenediaminetetraacetic acid and the like.

Examples of other containable compositions may include an antiseptic agent (such as ethylparaben, butylparaben, chlorphenesin, 2-phenoxyethanol, etc.); antiphlogistic (such as glycyrrhizinic acid derivatives, glycyrrhetic acid derivatives, salicylic acid derivatives, hinokitiol, zinc oxide, allantoin, etc.); a skin-whitening agent (such as placental extract, saxifrage extract, arbutin, etc.); various extracts (such as chestnut rose extract, phellodendron bark (cork tree bark), coptis rhizome, lithospermum, glycyrrhiza glabra, peony, swertia herb, birch, sage, loquat, carrot, aloe, okra, mallow, iris, grape, coix seed, rubus suavissimus leaf, sponge gourd, lily, prunus yedoensis leaf, saffron, cnidium rhizome, ginger, hypericum, restharrow, garlic, red pepper, citrus unshiu, Japanese angelica, green tea, seaweed, yeast, etc.); an activator (such as royal jelly, photosenstizer, cholesterol derivatives, etc.); a blood circulation accelerator (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc.) and the like.

Furthermore, the composition of the present disclosure may include, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

The viscosity of the composition of the present disclosure is less than 9,500 mPa·s, more preferably 9,000 mPa·s or less, more preferably 8,000 mPa·s or less, more preferably 7,000 mPa·s or less, more preferably 6,000 mPa·s or less, and further more preferably 5,000 mPa·s or less. By making the viscosity less than 10,000 mPa·s, the user can obtain a dewy feeling. The viscosity of the composition of the present disclosure is preferably 2,000 mPa·s or more, more preferably 3,000 mPa·s or more, and further more preferably 4,000 mPa·s or more. If the viscosity is less than 2,000 mPa·s, the composition will be too watery. The above-mentioned viscosity can be measured by a BL viscometer (rotor No. 3, rotation speed 12 rpm) at 30 °C.

Preferred embodiments will be described as the examples of the external oil-in-water emulsion for skin of the present disclosure. In the following description, (C) the hydrophilic surfactant will be featured on the basis of the above-mentioned description. However, the composition of the present disclosure is not limited to the following embodiments.

### [First embodiment]

In a first embodiment, an external oil-in-water emulsion composition for skin comprises a glycerin-derived surfactant among (C1) the nonionic surfactants as (C) the hydrophilic surfactant, and does not comprise a sorbitan-derived surfactant. The mass of the amphoteric surfactant is less than 0.5 times of the mass of (A) the silicone elastomer, or the content by percentage of (C2) the amphoteric surfactant is less than 0.5% by mass, and preferably, the composition does not comprise (C2) the amphoteric surfactant at a practical amount.

The glycerin-derived surfactant is preferably 0.2 part by mass or more, more preferably 0.3 part by mass or more, more preferably 0.4 part by mass or more, and further more preferably 0.5 part by mass or more with respect to 1 part by mass of (A) the silicone elastomer. If the glycerin-derived surfactant is less than 0.2 part by mass, effect on suppressing aggregation of the silicone elastomer will deteriorate. The glycerin-derived surfactant may be 4 parts by mass or less, 3 parts by mass or less, 2.5 parts by mass or less, or 2 parts by mass or less with respect to 1 part by mass of (A) the silicone elastomer. If the glycerin-derived surfactant exceeds 4 parts by mass, stickiness may occur upon application.

The content by percentage of the glycerin-derived surfactant is preferably 0.2% by mass or more, and more preferably 0.4% by mass or more with respect to the mass of the composition. If the content by percentage of the glycerin-derived surfactant is less than 0.2% by mass, stability of the silicone elastomer will deteriorate. The content by percentage of the glycerin-derived surfactant is preferably 5% by mass or less, more preferably 4% by mass or less, and further more preferably 3% by mass or less with respect to the mass of the composition. If the content by percentage of the glycerin-derived surfactant exceeds 5% by mass, stickiness may occur.

(C2) the amphoteric surfactant is preferably 1.5 parts by mass or less, more preferably 1 part by mass or less, more preferably 0.5 part by mass or less, and further more preferably 0 part by mass or less with respect to 1 part by mass of the glycerin-derived surfactant. If the amphoteric surfactant exceeds 1.5 parts by mass, (A) the silicone elastomer will aggregate easily.

The glycerin-derived surfactant is highly effective on dispersion stability and suppressing aggregation of the silicone elastomer. According to the first embodiment, stability of the silicone elastomer can be enhanced by a simpler formulation.

### [Second embodiment]

In a second embodiment, an external oil-in-water emulsion for skin comprises the glycerin-derived surfactant and the sorbitan-derived surfactant among (C1) the nonionic surfactant as (C) the hydrophilic surfactant. The mass of the amphoteric surfactant is less than 0.5 times of the mass of (A) the silicone elastomer, or the content by percentage of (C2) the amphoteric surfactant in the composition is less than 0.5% by mass, and the composition does not preferably comprise (C2) the amphoteric surfactant at a practical amount.

The glycerin-derived surfactant is preferably 0.2 part by mass or more, more preferably 0.25 part by mass or more, more preferably 0.3 part by mass or more, and further more preferably 0.35 part by mass or more with respect to 1 part by mass of (A) the silicone elastomer. If the glycerin-derived surfactant is less than 0.2 part by mass, effect on suppressing aggregation of the silicone elastomer will deteriorate. The glycerin-derived surfactant may be 4 parts by mass or less, 3 parts by mass or less, 2.5 parts by mass or less, or 2 parts by mass or less with respect to 1 part by mass of (A) the silicone elastomer. If the glycerin-derived surfactant exceeds 4 parts by mass, stickiness may occur upon application.

The sorbitan-derived surfactant is preferably 0.4 part by mass or more, more preferably 0.5 part by mass or more, more preferably 0.6 part by mass or more, and further more preferably 0.7 part by mass or more with respect to 1 part by mass of (A) the silicone elastomer. If the sorbitan-derived surfactant is less than 0.4 part by mass, effect on suppressing aggregation of the silicone elastomer will deteriorate. The sorbitan-derived surfactant may be 4 parts by mass or less, 3 parts by mass or less, 2.5 parts by mass or less, or 2 parts by mass or less with respect to 1 part by mass of (A) the silicone elastomer. If the sorbitan-derived surfactant exceeds 4 parts by mass, stickiness may occur upon application.

By using the glycerin-derived surfactant and the sorbitan-derived surfactant in combination, effect on suppressing aggregation of (A) the silicone elastomer can be further enhanced. According to the second embodiment, a composition with high stability can be obtained without increasing the viscosity.

### [Third embodiment]

In a third embodiment, an external oil-in-water emulsion composition for skin comprises the glycerin-derived surfactant and the sorbitan-derived surfactant among (C1) the nonionic surfactant as (C) the hydrophilic surfactant, and also comprises (C2) the amphoteric surfactant.

The glycerin-derived surfactant is preferably 0.2 part by mass or more, more preferably 0.25 part by mass or more, more preferably 0.3 part by mass or more, and further more preferably 0.35 part by mass or more with respect to 1 part by mass of (A) the silicone elastomer. If the glycerin-derived surfactant is less than 0.2 part by mass, effect on suppressing aggregation of the silicone elastomer will deteriorate. The glycerin-derived surfactant may be 4 parts by mass or less, 3 parts by mass or less, 2.5 parts by mass or less, or 2 parts by mass or less with respect to 1 part by mass of (A) the silicone elastomer. If the glycerin-derived surfactant exceeds 4 parts by mass, stickiness may occur upon application.

The sorbitan-derived surfactant is preferably 0.4 part by mass or more, more preferably 0.5 part by mass or more, more preferably 0.6 part by mass or more, and further more preferably 0.7 part by mass or more with respect to 1 part by mass of (A) the silicone elastomer. If the sorbitan-derived surfactant is less than 0.4 part by mass, effect on suppressing aggregation of the silicone elastomer will deteriorate. The sorbitan-derived surfactant may be 4 parts by mass or less, 3 parts by mass or less, 2.5 parts by mass or less, or 2 parts by mass or less with respect to 1 part by mass of (A) the silicone elastomer. If the sorbitan-derived surfactant exceeds 4 parts by mass, stickiness may occur upon application.

(C2) the amphoteric surfactant is preferably 0.15 part by mass or more, more preferably 0.2 part by mass or more, and further more preferably 0.3 part by mass or more with respect to 1 part by mass of the glycerin-derived surfactant. If (C2) the amphoteric surfactant is 0.15 part by mass or more, emulsification stability can be enhanced. (C2) the amphoteric surfactant is preferably 1.5 parts by mass or less, 1 part by mass or less, and further more preferably 0.7 part by mass or less with respect to 1 part by mass of the glycerin-derived surfactant. If the amphoteric surfactant exceeds 1.5 parts by mass, (A) the silicone elastomer will aggregate easily.

(C2) the amphoteric surfactant may be 0.05 part by mass or more, 0.1 part by mass or more, or 0.15 part by mass or more with respect to 1 part by mass of (A) the silicone elastomer. (C2) the amphoteric surfactant may be 0.5 part by mass or less, 0.45 part by less, 0.4 part by mass or less, 0.3 part by mass or less, or 0.2 part by mass or less with respect to 1 part by mass of (A) the silicone elastomer. Emulsion stability can be enhanced while aggregation of the silicone elastomer is suppressed if the amphoteric surfactant is within this range.

With respect to the mass ratio of (A) the silicone elastomer and (C2) the amphoteric surfactant in the composition of the present disclosure, the mass of (A) is preferably 2 or more, and more preferably 3 or more when the mass of (C2) is 1. With respect to the mass ratio of (A) the silicone elastomer and (C2) the amphoteric surfactant, the mass of (A) is preferably 7 or less, and more preferably 6.5 or less when the mass of (C2) is 1. Aggregation of the silicone elastomer can be suppressed if the mass ratio is within this range.

With respect to the mass ratio of (C1) the nonionic surfactant and (C2) the amphoteric surfactant in the composition of the present disclosure, the mass of (C1) is preferably 5 or more, and more preferably 6 or more when the mass of (C2) is 1. With respect to the mass ratio of (C1) the nonionic surfactant and (C2) the amphoteric surfactant, the mass of (C1) is preferably 13 or less, and more preferably 12 or less when the mass of (C2) is 1. When the mass of (C2) is 1, the mass of (C1) may be 10 or less.

The content by percentage of the amphoteric surfactant in the composition of the present disclosure is preferably 0.05% by mass or more, and more preferably 0.1% by mass or more with respect to the mass of the composition. If the content by percentage of the amphoteric surfactant is less than 0.05%, effect on suppressing aggregation of the silicone elastomer will deteriorate. The content by percentage of the amphoteric surfactant is preferably 0.5% by mass or less, and more preferably 0.4% by mass or less with respect to the mass of the composition. If the content by percentage of the amphoteric surfactant exceeds 0.5% by mass, the emulsified particles coalesce and thus emulsification stability will deteriorate.

In the third embodiment, aggregation of (A) the silicone elastomer is suppressed since the glycerin-derived surfactant and the sorbitan-derived surfactant are included. Furthermore, emulsification stability is also enhanced since (C2) the amphoteric surfactant is included.

In cases where the composition comprises the glycerin-derived surfactant, the glycerin-derived surfactant is preferably less than 0.2 part by mass, more preferably 0.1 part by mass or less, more preferably 0.05 part by mass or less, and further more preferably 0 part by mass with respect to 1 part by mass of (C2) the amphoteric surfactant. If the glycerin-derived surfactant is 0.2 part by mass or more, (A) the silicone elastomer will aggregate easily.

In the composition of the present disclosure, aggregation (coalescence) of the silicone elastomer can be suppressed even if it is low in viscosity, and thus the silicone elastomer can be dispersed stably.

The user can obtain comfortable and smooth feeling in use upon application to skin since the composition of the present disclosure comprises the silicone elastomer. Furthermore, the user can also obtain dewy feeling in use since the viscosity of the composition of the present disclosure is suitably low. Consequently, according to the composition of the present disclosure, the user can obtain dewy and smooth feeling in use. Furthermore, the silicone elastomer can also achieve effect on blurring pores and the like and occlusive effect by applying to skin.

Smooth feeling in use can be achieved while stickiness is suppressed by using the non-emulsifying type silicone elastomer (preferably a silicone elastomer in an indefinite-shaped gel-state).

In addition to a high dispersion stability of the silicone elastomer, aggregation (coalescence) of the emulsified particles can be suppressed and the emulsified particles can be dispersed stably in the composition of the present disclosure.

Next, methods for producing the external composition for skin of the present disclosure will be described. The external composition for skin of the present disclosure can be prepared according to generally known methods, without being limited to a specific method. For example, the external composition for skin can be prepared by adding an oil phase in which the silicone elastomer is dispersed to an aqueous phase while mixed with a hand mixer.

### EXAMPLES

Examples of the composition of the present disclosure will be described below. The composition of the present disclosure, however, is not limited to the following examples. The composition of the present disclosure is not limited to cosmetics, either. The unit of the content by percentage shown in the Tables is percent by mass. Each composition was evaluated for the evaluations in the Tables and the viscosity thereof was measured. Evaluation items shown in the Tables were evaluated on the basis of the following criteria. The viscosity was measured by a BL viscometer (rotor No. 3, rotation speed 12 rpm) at 30 °C.

The evaluation item "elastomer dispersion stability (immediately after sample preparation)" in the Tables indicates a result obtained by visually confirming dispersibility (aggregation property) of the silicone elastomer on a blackboard immediately after the sample was prepared.

The evaluation item "elastomer aggregation suppression (after rolling treatment)" indicates a result obtained by visually confirming dispersibility (aggregation property) of the silicone elastomer on a blackboard after rolling treatment. Rolling treatment is a treatment of which 30 ml of the composition is put into a 50 ml screw tube and the screw tube is rotated at a rotation speed 45 rpm for 4 hours at 25 °C.

### [Elastomer dispersion stability (immediately after sample preparation) and Elastomer aggregation suppression (after rolling treatment)]

A: Aggregation of the silicone elastomer was not observed.
B: Aggregation of the silicone elastomer was hardly observed.
C: Aggregation of the silicone elastomer was slightly observed.
D: Aggregation of the silicone elastomer was partially observed.
E: Aggregation of the silicone elastomer was observed.

### [Emulsification stability]

A: Coalescence of the emulsified particles was not observed.
B: The emulsified particles slightly coalesced over time.
C: The emulsified particles coalesced over time.
D: The oil component floated immediately after sample preparation.

### [Test examples 1 to 6]

Compositions were prepared by varying the content by percentage of the silicone elastomer.

In Test example 1, the component (A) silicone elastomer was not added. Smooth feeling in use was not achieved in the composition according to Test example 1.

In Test examples 2 to 6, the content by percentage of the component (A) silicone elastomer was varied in a range from 0.6% by mass to 1.2% by mass. The viscosities of all the compositions were 5,000 mPa·s or less, but the silicone elastomer was stably dispersed immediately after sample preparation. Accordingly, if the content by percentage of the silicone elastomer is at least 3% by mass or less, it is considered that the silicone elastomer can be stably dispersed by adding 0.2% by mass to 0.8% by mass of the component (B) and 0.5% by mass to 1.5% by mass of the component (C1).

In Test examples 2 and 6, the component (4), the glycerin-derived surfactant, was not added. In Test examples 2 and 6, aggregation of the elastomer increased after the rolling treatment compared to Test examples 3 to 5. Accordingly, it is considered that it is better to use the glycerin-derived surfactant and the sorbitan-derived surfactant in combination than using the sorbitan-derived surfactant alone for suppressing aggregation of the silicone elastomer. Furthermore, it is considered that the glycerin-derived surfactant has a high effect on suppressing aggregation of the silicone elastomer.

**[Table 1]**

| | Test Examples | | 1 | 2 | 3 | 4 | 5 | 6 |
|---|---|---|---|---|---|---|---|---|
| (1) | (A) Polysilicone-11 | | - | 0.6 | 0.6 | 0.84 | 1.2 | 1.2 |
| (2) | (B) PEG10-dimethicone *1 | | 0.5 | 0.5 | 0.5 | 0.5 | 0.5 | 0.5 |
| (3) | (C1) Polysorbate-20*2 | | 0.8 | 1 | 0.8 | 0.8 | 1.0 | 0.8 |
| (4) | (C1) PEG-60 glyceryl isostearate *3 | | 0.3 | - | 0.3 | 0.3 | 0.3 | - |
| (5) | (D) Dimethicone | | 4 | 8.4 | 8.4 | 10.2 | 12.8 | 12.5 |
| (6) | (D) Diphenylsiloxy phenyl trimethicone | | 1 | 1 | 1 | 1 | 1 | 1 |
| (7) | (E) Sodium Acrylate/Sodium Acryloyldimethyl Taurate Copolymer | | 1 | 1 | 1 | 1 | 1 | 1 |
| (8) | (E) Acrylates/C10-30 alkyl acrylate Crosspolymer | | 0.1 | 0.1 | 0.1 | 0.1 | 0.1 | 0.1 |
| (9) | (E) Xanthan gum | | 0.05 | 0.05 | 0.05 | 0.05 | 0.05 | 0.05 |
| (10) | (E) Succinoglycan | | 0.15 | 0.15 | 0.15 | 0.15 | 0.15 | 0.15 |
| (11) | (F) Bis-Diglyceryl polyacyladipate-2 | | 1 | - | 1 | 1 | 1 | - |
| (12) | (F) Pentaerythritol tetra(2-ethylhexanoate) | | 0.5 | - | 0.5 | 0.5 | 0.5 | - |
| (13) | (F) Isododecane | | - | 1 | - | - | - | - |
| (14) | (F) Deodorizing polybutene | | - | 1 | - | - | - | - |
| (15) | (F) Bis-PEG-18 methyl ether dimethyl silane | | 3 | 3 | 3 | 3 | 3 | 3 |
| (16) | (F) Glycerin | | 5 | 5 | 5 | 5 | 5 | 5 |
| (17) | (F) Dipropylene glycol | | 7 | 9 | 9 | 7 | 9 | 7 |
| (18) | (F) Polyethylene glycol | | 1 | 1 | 1 | 1 | 1 | 1 |
| (19) | (F) Hyaluronic acid | | 0.1 | 0.1 | 0.1 | 0.1 | 0.1 | 0.1 |
| (20) | (F) Water | | Balance | Balance | Balance | Balance | Balance | Balance |
| Total (% by mass) | | | 100 | 100 | 100 | 100 | 100 | 100 |
| Viscosity (mPa·s) | | | 3980 | 4110 | 4110 | 5250 | 4450 | 4450 |
| Evaluation | | Elastomer dispersion stability (immediately after sample preparation) | - | B | B | B | B | B |
| | | Elastomer aggregation suppression (after rolling treatment) | - | D | B | B | B | D |
| | | Emulsification stability | - | B | B | B | B | B |
| ^{∗}1: Silicone KF-6017P, Shin-Etsu Chemical Co., Ltd. | | | | | | | | |
| ^{∗}2: NIKKOL^{®} TL-10, Nikko Chemicals Co., Ltd. | | | | | | | | |
| ^{∗}3: EMALEX^{®} GWIS-160N, Nihon Emulsion Co., Ltd. | | | | | | | | |

### [Test examples 7 to 15]

In the formulation of Table 2 below, the lipophilic surfactants listed in Table 3 were added as the lipophilic surfactant of the component (2) to prepare compositions, respectively, and dispersion stability of the silicone elastomer was observed. The results are shown in Table 3. The component (B') in Test examples 7 and 9 to 12 is a polyglycerin-based surfactant. The component (B') in Test example 8 is a pentaerythritol-based surfactant. The component (B') in Test examples 13 to 15 is a polyethersilicone surfactant.

In Test examples 7 to 12, aggregation of the silicone elastomer was observed. On the other hand, in Test examples 13 to 15, aggregation of the silicone elastomer was hardly observed. Accordingly, it has been found that the silicone surfactant is preferable as the lipophilic surfactant for enhancing dispersibility of the silicone elastomer. The silicone elastomer used in Test examples 13 to 15 is a polyoxyethylene methylpolysiloxane copolymer that has a pendant-shaped structure in which the polyether side chains are branched from the silicone main chain.

**[Table 2]**

| | Test Examples | 7-15 |
|---|---|---|
| (1) | (A) Polysilicone-11 | 0.84 |
| (2) | (B') Lipophilic surfactant (refer to Table 3) | 0.8 |
| (3) | (C1) Polysorbate-20^{∗}2 | 0.8 |
| (4) | (C1) PEG-60 glyceryl isostearate ^{∗}3 | 0.3 |
| (5) | (D) Dimethicone | 10.2 |
| (6) | (D) Diphenylsiloxy phenyl trimethicone | 1 |
| (7) | (E) Sodium Acrylate/Sodium Acryloyldimethyl Taurate Copolymer | 1 |
| (8) | (E) Acrylates/C10-30 Alkyl Acrylate Crosspolymer | 0.1 |
| (9) | (E) Xanthan gum | 0.05 |
| (10) | (E) Succinoglycan | 0.15 |
| (11) | (F) Bis-diglyceryl polyacyladipate-2 | 1 |
| (12) | (F) Pentaerythritol tetra(2-ethylhexanoate) | 0.5 |
| (13) | (F) Bis-PEG-18 methyl ether dimethyl silane | 3 |
| (14) | (F) Glycerin | 5 |
| (15) | (F) Dipropylene glycol | 9 |
| (16) | (F) Hyaluronic acid | 0.1 |
| (17) | (F) Water | Balance |
| Total (% by mass) | | 100 |

**[Table 3]**

| | (B') Lipophilic surfactant | Elastomer dis persion stability | |
|---|---|---|---|
| | | Immediately after preparation | After rolling treatment |
| Test Example 7 | Polyglyceryl diisostearate^{∗}4 | E | E |
| Test Example 8 | Dipentaerythrityl tri-polyhydroxystearate ^{∗}5 | E | E |
| Test Example 9 | Bis-Butyldimethicone polyglyceryl-3^{∗}6 | D | E |
| Test Example 10 | Dimethicone/Polyglycerin-3 crosspolymer^{∗}7 | D | E |
| Test Example 11 | Polyglyceryl triisostearate^{∗}8 | E | E |
| Test Example 12 | Polyglyceryl-5 triisostearate^{∗}9 | D | E |
| Test Example 13 | PEG-10 Dimethicone^{∗}1 | B | B |
| Test Example 14 | PEG-9 Polydimethylsiloxyethyl dimethicone^{∗}10 | B | B |
| Test Example 15 | Lauryl PEG-9 Polydimethylsiloxyethyl dimethicone ^{∗}11 | B | B |
| ^{∗}4: WOGEL 18DV | | | |
| ^{∗}5: SALACOS^{®} WO-6, The Nisshin OilliO Group, Ltd. | | | |
| ^{∗}6: SILICONE KF-6019, Shin-Etsu Chemical Co., Ltd. | | | |
| ^{∗}7: KSG-710, Shin-Etsu Silicones Co., Ltd. | | | |
| ^{∗}8: COSMOL^{®} 43V, The Nisshin OilliO Group, Ltd. | | | |
| ^{∗}9: SUNSOFT^{®} A-193E-C, Taiyo Kagaku Co., Ltd. | | | |
| ^{∗}10: Silicone SC0938B, Shin-Etsu Chemical Co., Ltd. | | | |
| ^{∗}11: Silicone SC0928SL, Shin-Etsu Chemical Co., Ltd. | | | |

### [Test examples 16 to 26]

In Test examples 16 to 26, the content by percentage of the component (2) silicone surfactant was varied. In Test examples 16 to 18, the silicone surfactant was not added and the content by percentage of the component (6) hydrophilic surfactant was varied. The formulations and the results are shown in Tables 4 and 5.

In Test examples 16 to 18 in which the silicone surfactant is not included, aggregation of the silicone elastomer was partially observed immediately after sample preparation and aggregation of the elastomer was observed after the rolling treatment even if the content by percentage of the hydrophilic surfactant was varied. On the other hand, in Test examples 19 to 26 in which the silicone surfactant is included, dispersion stability of the silicone elastomer was improved even if the viscosities were low; such as 5,000 mPa·s or less in Test examples 19 to 21 and 24 to 26, and 8,500 mPa·s or less in Test example 22. Accordingly, it is considered that the content by percentage of the silicone surfactant is preferably 0.3% by mass or more, more preferably 0.5% by mass or more, and further more preferably 0.8% by mass or more. Furthermore, it is considered that the content by percentage of the silicone surfactant is preferably 2.5% by mass or less, more preferably 2% by mass or less, and further more preferably 1.5% by mass or less.

With respect to emulsification stability, the emulsified particles also coalesced and a stable oil-in-water composition could not be obtained in Test examples 16 to 18. On the other hand, emulsified state could be stabilized in Test examples 19 to 24 in which the silicone surfactant is included. In Test examples 25 and 26, oil floated and a stable emulsified state could not be achieved. It is considered that this is due to an effect of the kinds and/or the amount of the silicone surfactant. As described above, however, dispersion state of the silicone elastomer was good in Test examples 25 and 26.

In Test example 22, the viscosity was not intentionally increased compared to other test examples for suppressing aggregation of the silicone elastomer. It is considered that the viscosity was increased because a multiple emulsified state of W/O/W was made. In such case, it is considered that the content by percentage of the silicone surfactant is preferably 5% by mass or less, more preferably 4% by mass or less, and further more preferably 3% by mass or less.

**[Table 4]**

| | Test Examples | | 16 | 17 | 18 | 19 | 20 | 21 |
|---|---|---|---|---|---|---|---|---|
| (1) | (A) Polysilicone-11 | | 0.84 | 0.84 | 0.84 | 0.84 | 0.84 | 0.84 |
| (2) | (B) PEG10-Dimethicone ^{∗}1 | | - | - | - | 0.8 | 1 | 1.5 |
| (3) | (B) Lauryl PEG-9 polydimethylsiloxyethyl dimethicone^{∗}11 | | - | - | - | - | - | - |
| (4) | (B) PEG-9 Polydimethylsiloxyethyl dimethicone ^{∗}10 | | - | - | - | - | - | - |
| (5) | (C1) Polysorbate-20^{∗}2 | | 0.8 | 0.8 | 0.8 | 0.8 | 0.8 | 0.8 |
| (6) | (C1) PEG-60 glyceryl isostearate ^{∗}3 | | 0.3 | 0.5 | 0.8 | 0.3 | 0.3 | 0.3 |
| (7) | (C2) Lauryl dimethylaminoacetic acid betaine ^{∗}12 | | 0.15 | 0.15 | 0.15 | 0.15 | 0.15 | 0.15 |
| (8) | (D) Dimethicone | | 10.2 | 10.2 | 10.2 | 10.2 | 10.2 | 10.2 |
| (9) | (D) Diphenylsiloxy phenyl Trimethicone | | 1 | 1 | 1 | 1 | 1 | 1 |
| (10) | (E) Sodium Acrylate/Sodium Acryloyldimethyl Taurate Ccopolymer | | 1 | 1 | 1 | 1 | 1 | 1 |
| (11) | (E) Acrylates/C10-30 alkyl acrylate) Crosspolymer | | 0.1 | 0.1 | 0.1 | 0.1 | 0.1 | 0.1 |
| (12) | (E) Xanthan gum | | 0.05 | 0.05 | 0.05 | 0.05 | 0.05 | 0.05 |
| (13) | (E) Succinoglycan | | 0.15 | 0.15 | 0.15 | 0.15 | 0.15 | 0.15 |
| (14) | (F) Bis-diglyceryl polyacyladipate-2 | | 1 | 1 | 1 | 1 | 1 | 1 |
| (15) | (F) Pentaerythritol tetra(2-ethylhexanoate) | | 0.5 | 0.5 | 0.5 | 0.5 | 0.5 | 0.5 |
| (16) | (F) Bis-PEG-18 methyl ether dimethyl silane | | 3 | 3 | 3 | 3 | 3 | 3 |
| (17) | (F) Glycerin | | 5 | 5 | 5 | 5 | 5 | 5 |
| (18) | (F) Dipropylene glycol | | 9 | 9 | 9 | 9 | 7 | 9 |
| (19) | (F) Polyethylene glycol | | 1 | 1 | 1 | 1 | 1 | 1 |
| (20) | (F) Hyaluronic acid | | 0.1 | 0.1 | 0.1 | 0.1 | 0.1 | 0.1 |
| (21) | (F) Water | | Balance | Balance | Balance | Balance | Balance | Balance |
| Total (% by mass) | | | 100 | 100 | 100 | 100 | 100 | 100 |
| Viscosity (mPa·s) | | | 5160 | 4690 | 4660 | 4580 | 4560 | 4680 |
| Evaluation | | Elastomer dispersion stability (immediately after sample preparation) | D | D | D | A | A | A |
| | | Elastomer aggregation suppression (after rolling treatment) | E | E | E | B | B | B |
| | | Emulsification stability | D | D | C | A | A | A |
| ^{∗}12: NISSANANON^{®} BL-SF, NOF CORPORATION | | | | | | | | |

**[Table 5]**

| | Test Examples | 22 | 23 | 24 | 25 | 26 |
|---|---|---|---|---|---|---|
| (1) | (A) Polysilicone-11 | 0.84 | 0.84 | 0.84 | 0.84 | 0.84 |
| (2) | (B) PEG10-dimethicone ^{∗}1 (B) Lauryl PEG-9 | 3 | 0.5 | 0.7 | - | - |
| (3) | polydimethylsiloxyethyl dimethicone^{∗}11 | - | - | - | 0.5 | - |
| (4) | (B) PEG-9 Polydimethylsiloxyethyl dimethicone ^{∗}10 | - | - | - | - | 0.5 |
| (5) | (C1) Polysorbate-20^{∗}2 | 0.8 | 0.8 | 0.8 | 0.8 | 0.8 |
| (6) | (C1) PEG-60 glyceryl isostearate ^{∗}3 | 0.3 | 0.3 | 0.3 | 0.3 | 0.3 |
| (7) | (C2) Lauryl dimethylaminoacetic acid betaine ^{∗}12 | 0.15 | - | - | - | - |
| (8) | (D) Dimethicone | 10.2 | 10.2 | 10.2 | 10.2 | 10.2 |
| (9) | (D) Diphenylsiloxy phenyl trimethicone | 1 | 1 | 1 | 1 | 1 |
| (10) | (E) Sodium Acrylate/Sodium Acryloyldimethyl Taurate Copolymer | 1 | 1 | 1 | 1 | 1 |
| (11) | (E) Acrylates/C10-30 alkyl acrylate Crosspolymer | 0.1 | 0.1 | 0.1 | 0.1 | 0.1 |
| (12) | (E) Xanthan gum | 0.05 | 0.05 | 0.05 | 0.05 | 0.05 |
| (13) | (E) Succinoglycan | 0.15 | 0.15 | 0.15 | 0.15 | 0.15 |
| (14) | (F) Bis-diglyceryl polyacyladipate-2 | 1 | 1 | 1 | 1 | 1 |
| (15) | (F) Pentaerythritol tetra(2-ethylhexanoate) | 0.5 | 0.5 | 0.5 | 0.5 | 0.5 |
| (16) | (F) Bis-PEG-18 methyl ether Dimethyl Silane | 3 | 3 | 3 | 3 | 3 |
| (17) | (F) Glycerin | 5 | 5 | 5 | 5 | 5 |
| (18) | (F) Dipropylene glycol | 7 | 9 | 9 | 9 | 9 |
| (19) | (F) Polyethylene glycol | 1 | | | | |
| (20) | (F) Hyaluronic acid | 0.1 | 0.1 | 0.1 | 0.1 | 0.1 |
| (21) | (F) Water | Balance | Balance | Balance | Balance | Balance |
| Total (% by mass) | | 100 | 100 | 100 | 100 | 100 |
| Viscosity (mPa·s) | | 8440 | 4790 | 4410 | 4530 | 3960 |
| Evaluation | Elastomer dispersion stability (immediately after sample preparation) | A | B | A | B | B |
| | Elastomer aggregation suppression (after rolling treatment) | B | B | B | B | B |
| | Emulsification stability | A | A | A | D | D |

### [Test examples 27 to 37]

In Test examples 27 to 37, the content by percentage of the hydrophilic surfactant was varied. The formulations and the results are shown in Tables 6 and 7. In any of the test examples, the viscosities were low such as 6,000 mPa·s or less, preferably 5,000 mPa·s or less, but dispersion stability of the silicone elastomer immediately after sample preparation was good.

Referring to other test examples at the same time, aggregation of the elastomer is suppressed in general when the amount of (B) the silicone surfactant is large. Thus, it is considered that the silicone surfactant has a high effect on suppressing aggregation of the elastomer.

In cases where the component (4), the glycerin-derived surfactant, and/or the component (5), the sterol-derived surfactant, are/is added in addition to the component (3), the sorbitan-derived surfactant, among the hydrophilic nonionic surfactants, effect on suppressing aggregation of the elastomer after rolling treatment was enhanced. Accordingly, it is considered that, as the hydrophilic nonionic surfactant, the combination of the sorbitan-derived surfactant and the glycerin-derived surfactant is more effective than the sorbitan-derived surfactant alone for enhancing effect on suppressing aggregation of the elastomer. It has also been revealed that the hydrophilic nonionic surfactant other than the sorbitan-based and the glycerin-based surfactants can be added. Furthermore, referring to other test examples at the same time, it is considered that the glycerin-derived surfactant has a high effect on suppressing aggregation of the elastomer. In particular, the component(s) (4) and/or (5) and/is added at preferably 0.4% by mass or more, and more preferably 0.5% by mass or more.

Effect on suppressing aggregation of the elastomer could be further enhanced when the component (6), the amphoteric surfactant, was used together. Accordingly, it is considered that the combination of the nonionic surfactant and the amphoteric surfactant is effective for suppressing aggregation of the elastomer. Furthermore, emulsification stability tends to be enhanced when the component (6) is added, and it is considered that the amphoteric surfactant has a high effect on emulsification stability. It is considered that the content by percentage of the amphoteric surfactant is preferably 0.1% by mass or more, more preferably 0.15% by mass or more, and further more preferably 0.2% by mass or more.

Significant difference was not observed for dispersibility of the silicone elastomer even if the kind of (E) the oily component was varied. Accordingly, it is considered that dispersibility of the silicone elastomer does not depend on the kind of the oily component.

There was no problem for emulsification stability in any of Test examples 27 to 37.

**[Table 6]**

| | Test Examples | | 27 | 28 | 29 | 30 | 31 | 32 |
|---|---|---|---|---|---|---|---|---|
| (1) | (A) Polysilicone-11 | | 1.2 | 0.84 | 0.84 | 0.84 | 0.84 | 0.84 |
| (2) | (B) PEG10-dimethicone ^{∗}1 | | 0.5 | 0.5 | 0.7 | 0.5 | 0.5 | 0.5 |
| (3) | (C1) Polysorbate-20^{∗}2 | | 0.8 | 0.8 | 0.7 | 0.8 | 0.8 | 1 |
| (4) | (C1) PEG-60 glyceryl isostearate ^{∗}3 | | - | - | 0.25 | 0.3 | 0.5 | 0.3 |
| (5) | (C1) PEG-30 phytosterol^{∗}13 | | - | - | - | - | - | - |
| (6) | (C2) Lauryl dimethylaminoacetic acid betaine ^{∗}12 | | - | - | - | - | - | - |
| (7) | (D) Dimethicone | | 12.5 | 10.2 | 10.2 | 10.2 | 10.2 | 10.2 |
| (8) | (D) Diphenylsiloxy phenyl trimethicone | | 1 | 1 | 1 | 1 | 1 | 1 |
| (9) | (E) Sodium Acrylate/Sodium Acryloyldimethyl Taurate Copolymer | | 1 | 1 | 1 | 1 | 1 | 1 |
| (10) | (E) Acrylates/C10-30 Alkyl Acrylate | | 0.1 | 0.1 | 0.1 | 0.1 | 0.1 | 0.1 |
| | Crosspolymer | | | | | | | |
| (11) | (E) Xanthan gum | | 0.05 | 0.05 | 0.05 | 0.05 | 0.05 | 0.05 |
| (12) | (E) Succinoglycan | | 0.15 | 0.15 | 0.15 | 0.15 | 0.15 | 0.15 |
| (13) | (F) Bis-diglyceryl polyacyladipate-2 | | - | - | 1 | 1 | 1 | 1 |
| (14) | (F) Pentaerythritol tetra(2-ethylhexanoate) | | - | - | 0.5 | 0.5 | 0.5 | 0.5 |
| (15) | (F) Isostearic Acid | | - | - | - | - | - | - |
| (16) | (F) Isododecane | | - | 1 | - | - | - | - |
| (17) | (F) Hydrogenated polyisobutene (Molecular weight: 1,000)^{∗}14 | | - | - | - | - | - | - |
| (18) | (F) Hydrogenated polyisobutene (Molecular weight: 2,850)^{∗}15 Bis-PEG-18 ether | | - | 0.5 | - | - | - | - |
| (19) | (F) methyl dimethyl silane | | 3 | 3 | 3 | 3 | 3 | 3 |
| (20) | (F) Glycerin | | 5 | 5 | 5 | 5 | 5 | 5 |
| (21) | (F) Dipropylene glycol | | 7 | 9 | 9 | 7 | 9 | 9 |
| (22) | (F) Polyethylene glycol | | 1 | 1 | 1 | 1 | 1 | 1 |
| (23) | (F) Hyaluronic acid | | 0.1 | 0.1 | 0.1 | 0.1 | 0.1 | 0.1 |
| (24) | (F) Water | | Balance | Balance | Balance | Balance | Balance | Balance |
| Total (% by mass) | | | 100 | 100 | 100 | 100 | 100 | 100 |
| Viscosity (mPa·s) | | | 4450 | 4250 | 5250 | 5250 | 4490 | 4300 |
| Evaluation | | Elastomer dispersion stability (immediately after sample preparation) Elastomer aggregation | B | B | A | B | A | A |
| | | suppression (after rolling treatment) | D | D | B | B | B | B |
| | | Emulsification stability | B | B | A | B | A | B |
| ^{∗}13: NIKKOL^{®} BPS-30 Nikko Chemicals Co., Ltd. | | | | | | | | |
| ^{∗}14: Deodorizing polybutene P | | | | | | | | |
| ^{∗}15: Deodorizing polybutene 200SH | | | | | | | | |

**[Table 7]**

| | Test Examples | | 33 | 34 | 35 | 36 | 37 |
|---|---|---|---|---|---|---|---|
| (1) | (A) Polysilicone-11 | | 0.6 | 0.84 | 0.84 | 0.6 | 0.84 |
| (2) | (B) PEG10-dimethicone ^{∗}1 | | 0.5 | 0.8 | 1 | 0.5 | 0.5 |
| (3) | (C1) Polysorbate-20^{∗}2 | | 0.8 | 0.8 | 1.5 | 1 | 0.8 |
| (4) | (C1) PEG-60 glyceryl isostearate ^{∗}3 | | 0.3 | 0.3 | 1 | | 0.3 |
| (5) | (C1) PEG-30 phytosterol^{∗}13 | | | | | | 0.2 |
| (6) | (C2) Lauryl dimethylaminoacetic acid betaine ^{∗}12 | | 0.15 | 0.15 | 0.31 | | |
| (7) | (D) Dimethicone | | 8.4 | 10.2 | 9.86 | 8.4 | 10.2 |
| (8) | (D) Diphenylsiloxy phenyl trimethicone | | 1 | 1 | 1 | 1 | 1 |
| (9) | (E) Sodium Acrylate/Sodium Acryloyldimethyl Taurate Copolymer | | 1 | 1 | 1 | 1 | 1 |
| (10) | (E) Acrylates/C10-30 Alkyl Acrylate Crosspolymer | | 0.1 | 0.1 | 0.1 | 0.1 | 0.1 |
| (11) | (E) Xanthan gum | | 0.05 | 0.05 | 0.05 | 0.05 | 0.05 |
| (12) | (E) Succinoglycan | | 0.15 | 0.15 | 0.15 | 0.15 | 0.15 |
| (13) | (F) Bis-diglyceryl polyacyladipate-2 | | 1 | 1 | 1 | | 1 |
| (14) | (F) Pentaerythritol tetra(2-ethylhexanoate) | | 0.5 | 0.5 | 0.5 | - | 0.5 |
| (15) | (F) Isostearic acid | | 0.23 | 0.23 | 0.45 | - | - |
| (16) | (F) Isododecane | | - | - | - | 1 | - |
| (17) | (F) Hydrogenated polyisobutene (Molecular weight: 1,000)^{∗}14 | | - | - | - | 1 | - |
| (18) | (F) Hydrogenated polyisobutene (Molecular weight: 2,850)^{∗}15 | | - | - | - | - | - |
| (19) | (F) Bis-PEG-18 methyl ether dimethyl silane | | 3 | 3 | 3 | 3 | 3 |
| (20) | (F) Glycerin | | 5 | 5 | 5 | 5 | 5 |
| (21) | (F) Dipropylene glycol | | 9 | 9 | 9 | 7 | 9 |
| (22) | (F) Polyethylene glycol | | 1 | 1 | 1 | 1 | 1 |
| (23) | (F) Hyaluronic acid | | 0.1 | 0.1 | 0.1 | 0.1 | 0.1 |
| (24) | (F) Water | | Balance | Balance | Balance | Balance | Balance |
| Total (% by mass) | | | 100 | 100 | 100 | 100 | 100 |
| Viscosity (mPa·s) | | | 4360 | 4580 | 5760 | 4110 | 4510 |
| Evaluation | | Elastomer dispersion stability (immediately after sample preparation) | B | A | A | B | B |
| | | Elastomer aggregation suppression (after rolling treatment) | B | B | B | D | B |
| | | Emulsification stability | A | A | A | B | B |

Focusing on the mass ratio of (A) the silicone elastomer and (B) the silicone surfactant, the above-mentioned test examples show that the mass of (A) is 0.56 times of the mass of (B) in Test example 21 that has the smallest mass ratio of (A) with respect to (B). In Test example 5 that has the largest mass ratio of (A) with respect to (B), the mass of (A) is 2.4 times of the mass of (B). In Test examples 19 and 34 (of the same formulations) that achieved the best result, the mass of (A) is 1.05 times of the mass of (B). Accordingly, it is considered that the mass of (A) is preferably 0.4 or more, more preferably 0.5 or more, and further more preferably 0.8 or more when the mass of (B) is 1. It is considered that the mass of (A) is preferably 3 or less, and more preferably 2 or less when the mass of (B) is 1.

Focusing on the mass ratio of (A) the silicone elastomer and (C) the hydrophilic surfactant (C1 + C2), the above-mentioned test examples show that the mass of (A) is 0.30 times of the mass of (C) in Test example 35 that has the smallest mass ratio of (A) with respect to (C). In the below-mentioned test example 39 that has the largest mass ratio of (A) with respect to (C), the mass of (A) is 1.68 times of the mass of (C). In Test examples 19 and 34 (of the same formulations) that achieved the best results, the mass of (A) is 0.67 times of the mass of (C1). Accordingly, it is considered that the mass of (A) is preferably 0.2 or more, more preferably 0.3 or more, and further more preferably 0.5 or more when the mass of (C) is 1. It is considered that the mass of (A) is preferably 2 or less, and more preferably 1.5 or less when the mass of (C) is 1.

Focusing on the mass ratio of the sorbitan-derived surfactant and the glycerin-derived surfactant, the above-mentioned test examples show that the mass of the sorbitan type is 1.5 times of the mass of the glycerin type in Test example 35 that has the smallest mass ratio of the sorbitan type with respect to the glycerin type. In Test example 5 that has the largest mass ratio of the sorbitan type with respect to the glycerin type, the mass of the sorbitan type is 3.3 times of the glycerin type. In Test examples 19 and 34 (of the same formulations) that achieved the best result, the mass of the sorbitan type is 2.7 times of the mass of the glycerin type. Accordingly, it is considered that the mass of the sorbitan type is preferably 1 or more, more preferably 1.3 or more, and further more preferably 2 or more when the mass of the glycerin type is 1. It is considered that the mass of the sorbitan type is 5 or less, more preferably 4 or less, and further more preferably 3.5 or less when the mass of the glycerin type is 1.

Focusing on the mass ratio of (A) the silicone elastomer and (C1) the nonionic surfactant, the above-mentioned test examples show that the mass of (A) is 0.34 times of the mass of (C1) in Test example 35 of which the mass ratio of (A) with respect to (C1) is the smallest. In the below-mentioned test example 39 that has the largest mass ratio of (A) with respect to (C1), the mass of (A) is 1.68 times of the mass of (C1). In Test examples 19 and 34 (of the same formulations) that achieved the best result, the mass of (A) is 0.76 times of the mass of (C1). Accordingly, it is considered that the mass of (A) is preferably 0.2 or more, and more preferably 0.5 or more when the mass of (C1) is 1. It is considered that the mass of (A) is preferably 2 or less, and more preferably 1.5 or less when the mass of (C1) is 1.

Focusing on the mass ratio of (A) the silicone elastomer and (C2) the amphoteric surfactant in cases where the mass of (C2) the amphoteric surfactant is less than 0.5 times of the mass of (A) the silicone elastomer (or in cases where (C2) is less than 0.5% by mass with respect to the mass of the composition), the above-mentioned test examples show that the mass of (A) is 2.7 times of the mass of (C2) in Test example 35 that has the smallest mass ratio of (A) with respect to (C2). In Test examples 19 to 22 and 34 that have the largest mass ratio of (A) with respect to (C2), the mass of (A) is 5.6 times of the mass of (C2). In Test examples 19 and 34 (of the same formulations) that achieved the best result, the mass of (A) is 5.6 times of the mass of (C2). Accordingly, it is considered that the mass of (A) is preferably 2 or more, and more preferably 3 or more when the mass of (C2) is 1. It is considered that the mass of (A) is preferably 7 or less, and more preferably 6.5 or less when the mass of (C2) is 1.

Focusing on the mass ratio of (C1) the nonionic surfactant and (C2) the amphoteric surfactant in cases where the mass of (C2) the amphoteric surfactant is less than 0.5 times of the mass of (A) the silicone elastomer (or in cases where (C2) is less than 0.5% by mass with respect to the mass of the composition), the above-mentioned test examples show that the mass of (C1) is 7.3 times of the mass of (C2) in Test examples 19 to 22 and the like that have the smallest mass ratio of (C1) with respect to (C2). In Test example 35 that has the largest mass ratio of (C1) with respect to (C2), the mass of (C1) is 8.1 times of the mass of (C2). In Test examples 19 and 34 (of the same formulations) that achieved the best result, the mass of (C1) is 7.3 times of the mass of (C2). Accordingly, it is considered that the mass of (C1) is preferably 5 or more, and more preferably 6 or more when the mass of (C2) is 1. It is considered that the mass of (C1) is preferably 13 or less, more preferably 12 or less, and further more preferably 10 or less when the mass of (C2) is 1.

Focusing on the mass ratio of (A) the silicone elastomer and the glycerin type surfactant in cases where the mass of (C2) the amphoteric surfactant is less than 0.5 times of the mass of (A) the silicone elastomer (or in cases where (C2) is less than 0.5% by mass with respect to the mass of the composition), the above-mentioned test examples show that the mass of the glycerin type surfactant is 0.25 times of the mass of (A) in Test example 5 that has the smallest mass ratio of the glycerin type surfactant with respect to (A). In Test example 35 that has the largest mass ratio of the glycerin type surfactant with respect to (A), the mass of the glycerin type surfactant is 1.2 times of the mass of (A). In Test examples 19 and 34 (of the same formulations) that achieved the best result, the mass of the glycerin type surfactant is 0.36 times of the mass of (A). Accordingly, it is considered that the mass of the glycerin type surfactant is preferably 0.2 or more, and more preferably 0.3 or more when the mass of (A) is 1. It is considered that the mass of the glycerin type surfactant is preferably 4 or less, and more preferably 3 or less when the mass of (A) is 1.

Focusing on the mass ratio of the glycerin type surfactant and (C2) the amphoteric surfactant in cases where the mass of (C2) the amphoteric surfactant is less than 0.5 times of the mass of (A) the silicone elastomer (or in cases where (C2) is less than 0.5% by mass with respect to the mass of the composition), the above-mentioned test examples show that the mass of (C2) is 0.3 times of the mass of the glycerin type surfactant in Test example 35 that has the smallest mass ratio of (C2) with respect to the glycerin type surfactant. In Test examples 19 to 22 and 33 to 34 that have the largest mass ratio of (C2) with respect to the glycerin type surfactant, the mass of (C2) is 0.5 times of the mass of the glycerin type surfactant. Accordingly, it is considered that the mass of (C2) is preferably 0.15 or more, more preferably 0.2 or more, and further more preferably 0.25 or more when the mass of the glycerin type surfactant is 1. It is considered that the mass of (C2) is preferably 1.5 or less, and more preferably 1 or less when the mass of the glycerin type surfactant is 1.

The above-mentioned test examples show that good results were achieved when the total amount of the silicone oil was 9.4% by mass to 13.8% by mass. Accordingly, it is considered that the content by percentage of the silicone oil is 8% by mass or more, and preferably 9% by mass or more. Furthermore, it is considered that the content by percentage of the silicone oil is 15% by mass or less, and preferably 14% by mass or less.

The above-mentioned test examples show that good results were achieved when the thickener was at least taurate-based synthetic polymer and/or acrylate-based synthetic polymer.

In the compositions of the above-mentioned test examples, the average particle size of the silicone elastomer was 25 µm to 50 µm. The average particle size was measured by microscopic observation.

### [Test examples 38 to 41]

In Test examples 38 to 41, the sorbitan-derived surfactant was not added but the component (3), the glycerin-derived surfactant, was added as the hydrophilic nonionic surfactant to prepare the compositions and the content by percentage of the component (3) was varied. The amphoteric surfactant was not added. The formulations and the results are shown in Table 8.

In Test example 38 in which the component (C) nonionic surfactant was not added, dispersion stability of the silicone elastomer immediately after sample preparation was good, but aggregation of the elastomer increased by rolling treatment. Furthermore, the viscosity of the composition of Test example 38 was more than 6,000 mPa·s. On the other hand, in Test examples 39 to 41 in which the glycerin-derived nonionic surfactant was added, the viscosity was 5,000 mPa·s or less, and dispersion stability of the silicone elastomer immediately after sample preparation was good, too. Furthermore, aggregation of the elastomer after rolling treatment was improved compared to Test example 38. Accordingly, it is revealed that the glycerin-derived surfactant alone can enhance effect on suppressing aggregation of the elastomer.

In Test examples in which the glycerin-derived surfactant was not added, but the sorbitan-derived surfactant was added as the hydrophilic nonionic surfactant (for example Test examples 2, 27 and 28), aggregation of the silicone elastomer after rolling treatment increased. In Test examples 39 to 41, however, aggregation of the silicone elastomer after rolling treatment was suppressed. Therefore, it is considered that the glycerin-derived surfactant has a higher effect on suppressing aggregation of the silicone elastomer than the sorbitan-derived surfactant.

In Test examples 39 to 41, the evaluation of suppressing aggregation after rolling treatment was lowered for 2 levels from the evaluation of dispersion stability immediately after sample preparation (for example, from A to C). In Test examples in which both of the glycerin-derived surfactant and the sorbitan-derived surfactant were added (for example, Test examples 16 to 24, 29 to 35 and 37), lowering of the evaluation from immediately after sample preparation to after the rolling treatment was 1 level or less (for example, from A to B, or from B to B). Accordingly, it is preferred to add both of the glycerin-derived surfactant and the sorbitan-derived surfactant for enhancing effect on suppressing aggregation of the silicone elastomer.

The glycerin-derived surfactant is preferably 0.2 part by mass or more, more preferably 0.3 part by mass or more, more preferably 0.4 part by mass or more, and further more preferably 0.5 part by mass or more with respect to 1 part by mass of the silicone elastomer. If the glycerin-derived surfactant is less than 0.2 part by mass, effect on suppressing aggregation of the silicone elastomer will deteriorate. The glycerin-derived surfactant can be 4 parts by mass or less, 3 parts by mass or less, 2.5 parts by mass or less, or 2 parts by mass or less with respect to 1 part by mass of (A) the silicone elastomer. If the glycerin-derived surfactant exceeds 4 parts by mass, stickiness may occur upon application.

In order to enhance stability of the silicone elastomer, the content by percentage of the glycerin-derived surfactant is preferably 0.2% by mass or more, and more preferably 0.3% by mass or more with respect to the mass of the composition.

**[Table 8]**

| | Test Examples | | 38 | 39 | 40 | 41 |
|---|---|---|---|---|---|---|
| (1) | (A) Polysilicone-11 | | 0.84 | 0.84 | 0.84 | 0.84 |
| (2) | (B) PEG10-dimethicone ^{∗}1 | | 0.8 | 0.8 | 0.8 | 0.8 |
| (3) | (C1) PEG-60 glyceryl isostearate ^{∗}3 | | | 0.5 | 0.8 | 1 |
| (4) | (D) Dimethicone | | 9.86 | 9.86 | 9.86 | 9.86 |
| (5) | (D) Diphenylsiloxy phenyl trimethicone | | 1 | 1 | 1 | 1 |
| (6) | (E) Sodium Acrylate/Sodium Acryloyldimethyl Taurate Copolymer | | 1 | 1 | 1 | 1 |
| (7) | (E) Acrylates/C10-30 Alkyl Acrylate Crosspolymer r | | 0.1 | 0.1 | 0.1 | 0.1 |
| (8) | (E) Xanthan gum | | 0.05 | 0.05 | 0.05 | 0.05 |
| (9) | (E) Succinoglycan | | 0.15 | 0.15 | 0.15 | 0.15 |
| (10) | (F) Bis-diglyceryl polyacyladipate-2 | | 1 | 1 | 1 | 1 |
| (11) | (F) Pentaerythritol tetra(2-ethylhexanoate) | | 0.5 | 0.5 | 0.5 | 0.5 |
| (12) | (F) Bis-PEG-18 methyl ether dimethyl silane | | 3 | 3 | 3 | 3 |
| (13) | (F) Glycerin | | 5 | 5 | 5 | 5 |
| (14) | (F) Dipropylene glycol | | 9 | 9 | 7 | 9 |
| (15) | (F) Polyethylene glycol | | 1 | 1 | 1 | 1 |
| (16) | (F) Sodium hyaluronate | | | 0.1 | 0.1 | 0.1 |
| (17) | (F) Water | | Balance | Balance | Balance | Balance |
| Total (% by mass) | | | 100 | 100 | 100 | 100 |
| Viscosity (mPa· s) | | | 6580 | 4430 | 4130 | 4360 |
| Evaluation | | Elastomer dispersion stability (immediately after sample preparation) | B | A | A | A |
| | | Elastomer aggregation suppression (after rolling treatment) | E | C | C | C |

### [Test examples 42 to 48]

In Test examples 42 to 48, the content by percentage of the component (5), the amphoteric surfactant, was varied. In Test examples 42 and 43, the nonionic surfactant was not added. In Test examples 44 and 45, the components (3) and (4), the nonionic surfactants, were added. In Test examples 46 to 48, the component (4), the glycerin-derived surfactant, was added as the nonionic surfactant, but the component (4), the sorbitan-derived surfactant, was not added. The formulations and the results are shown in Tables 9 and 10.

Among Test examples 42 and 43 that do not comprise the nonionic surfactant, the evaluation after rolling treatment was largely lowered from the evaluation immediately after sample preparation in Test example 42 of which the content by percentage of the amphoteric surfactant was lower. On the other hand, in Test example 43 of which the content by percentage of the amphoteric surfactant was higher, aggregation by rolling treatment could be suppressed. Furthermore, dispersion stability immediately after sample preparation was enhanced in Test example 43 compared to Test example 42. Furthermore, the viscosity of Test example 43 was lowered compared to Test example 42. Accordingly, it is revealed that dispersion stability and effect on suppressing aggregation of the silicone elastomer can be enhanced by the amphoteric surfactant alone even if the nonionic surfactant is not used.

In cases where the nonionic surfactant is not included, the amphoteric surfactant is preferably 0.65 part by mass or more, more preferably 0.7 part by mass or more, more preferably 0.75 part by mass or more, more preferably 0.8 part by mass or more, and further more preferably 0.85 part by mass or more with respect to 1 part by mass of the silicone elastomer. If the amphoteric surfactant is less than 0.65 part by mass, effect on suppressing aggregation of the silicone elastomer will deteriorate. The amphoteric surfactant is preferably 4 parts by mass or less, and more preferably 3 parts by mass or less with respect to 1 part by mass of the silicone elastomer.

In order to enhance stability of the silicone elastomer, the content by percentage of the amphoteric surfactant is preferably 0.6% by mass or more, and more preferably 0.7% by mass or more with respect to the mass of the composition.

In Test examples 44 to 48, dispersion stability immediately after sample preparation was good in all test examples, but the silicone elastomer aggregated by rolling treatment. On the other hand, in Test examples 19 to 22 and 33 to 35 that comprise both of the nonionic surfactant and the amphoteric surfactant, such results were not observed. Accordingly, it is considered that the elastomer tends to aggregate easily as the amount of the amphoteric surfactant becomes relatively larger with respect to that of the other components.

When Test examples 44 to 45 and Test examples 46 to 48 are compared, aggregation of the silicone elastomer occurred in Test examples 46 to 48 that do not comprise the sorbitan-derived surfactant. Aggregation of the silicone elastomer could be suppressed in Test example 43 that does not comprise the glycerin-derived surfactant. Accordingly, it is considered that the component that deteriorates effect on suppressing aggregation when it is combined with the amphoteric surfactant is the glycerin-derived surfactant. However, as shown in Test examples 19 to 22 and 33 to 35, emulsification stability can be suppressed while aggregation of the silicone elastomer can be suppressed by the combination of the amphoteric surfactant and the glycerin-derived surfactant.

In cases where the composition comprises the amphoteric surfactant at 0.5 part by mass or more with respect to 1 part by mass of the silicone elastomer (or in cases where the content by percentage of the amphoteric surfactant is 0.5% by mass or more with respect to the mass of the composition), it is considered that the glycerin-derived surfactant is preferably less than 0.2 part by mass, more preferably 0.1 part by mass or less, more preferably 0.05 part by mass or less, and further more preferably 0 part by mass with respect to 1 part by mass of the amphoteric surfactant.

In order to enhance stability of the silicone elastomer, the content by percentage of the amphoteric surfactant is preferably 0.6% by mass or more, more preferably 0.7% by mass or more, and further more preferably 0.8% by mass or more with respect to the mass of the composition.

**[Table 9]**

| | Test Examples | | 42 | 43 | 44 | 45 |
|---|---|---|---|---|---|---|
| (1) | (A) Polysilicone-11 | | 0.84 | 0.84 | 0.84 | 0.84 |
| (2) | (B) PEG10-dimethicone ^{∗}1 | | 0.8 | 0.8 | 0.8 | 0.8 |
| (3) | (C1) Polysorbate-20^{∗}2 | | - | - | 0.8 | 0.8 |
| (4) | (C1) PEG-60 glyceryl isostearate ^{∗}3 | | - | - | 0.3 | 0.3 |
| (5) | (C2) Lauryl dimethylaminoacetic acid betaine ^{∗}12 | | 0.5 | 1 | 0.6 | 1 |
| (6) | (D) Dimethicone | | 9.86 | 9.86 | 9.86 | 9.86 |
| (7) | (D) Diphenylsiloxy phenyl trimethicone | | 1 | 1 | 1 | 1 |
| (8) | (E) Sodium Acrylate/Sodium Acryloyldimethyl Taurate Copolymer | | 1 | 1 | 1 | 1 |
| (9) | (E) Acrylates/C10-30 Alkyl Acrylate Crosspolymer | | 0.1 | 0.1 | 0.1 | 0.1 |
| (10) | (E) Xanthan gum | | 0.05 | 0.05 | 0.05 | 0.05 |
| (11) | (E) Succinoglycan | | 0.15 | 0.15 | 0.15 | 0.15 |
| (12) | (F) Bis-diglyceryl polyacyladipate-2 | | 1 | 1 | 1 | 1 |
| (13) | (F) Pentaerythritol tetra(2-ethylhexanoate) | | 0.5 | 0.5 | 0.5 | 0.5 |
| (14) | (F) Bis-diglyceryl polyacyladipate-2 | | 3 | 3 | 3 | 3 |
| (15) | (F) Glycerin | | 5 | 5 | 5 | 5 |
| (16) | (F) Dipropylene glycol | | 9 | 9 | 7 | 9 |
| (17) | (F) Polyethylene glycol | | 1 | 1 | 1 | 1 |
| (18) | (F) Sodium hyaluronate | | 0.1 | 0.1 | 0.1 | 0.1 |
| (19) | (F) Water | | Balance | Balance | Balance | Balance |
| Total (% by mass) | | | 100 | 100 | 100 | 100 |
| Viscosity (mPa· s) | | | 7350 | 6040 | 4250 | 4660 |
| Evaluation | | Elastomer dispersion stability (immediately after sample preparation) | B | A | A | A |
| | | Elastomer aggregation suppression (after rolling treatment) | E | B | E | E |

**[Table 10]**

| | Test Examples | | 46 | 47 | 48 |
|---|---|---|---|---|---|
| (1) | (A) Polysilicone-11 | | 0.84 | 0.84 | 0.84 |
| (2) | (B) PEG10-dimethicone ^{∗}1 | | 0.8 | 0.8 | 0.8 |
| (3) | (C1) Polysorbate-20^{∗}2 | | - | - | - |
| (4) | (C1) PEG-60 glyceryl isostearate ^{∗}3 | | 0.3 | 0.3 | 0.3 |
| (5) | (C2) Lauryl dimethylaminoacetic acid betaine ^{∗}12 | | 0.5 | 1 | 1.5 |
| (6) | (D) Dimethicone | | 9.86 | 9.86 | 9.86 |
| (7) | (D) Diphenylsiloxy phenyl trimethicone | | 1 | 1 | 1 |
| (8) | (E) Sodium Acrylate/Sodium Acryloyldimethyl Taurate Copolymer | | 1 | 1 | 1 |
| (9) | (E) Acrylates/C10-30 Alkyl Acrylate Crosspolymer | | 0.1 | 0.1 | 0.1 |
| (10) | (E) Xanthan gum | | 0.05 | 0.05 | 0.05 |
| (11) | (E) Succinoglycan | | 0.15 | 0.15 | 0.15 |
| (12) | (F) Bis-Diglyceryl polyacyladipate-2 | | 1 | 1 | 1 |
| (13) | (F) Pentaerythritol tetra(2-ethylhexanoate) | | 0.5 | 0.5 | 0.5 |
| (14) | (F) Bis-PEG-18 methyl ether dimethyl silane | | 3 | 3 | 3 |
| (15) | (F) Glycerin | | 5 | 5 | 5 |
| (16) | (F) Dipropylene glycol | | 9 | 7 | 9 |
| (17) | (F) Polyethylene glycol | | 1 | 1 | 1 |
| (18) | (F) Sodium hyaluronate | | 0.1 | 0.1 | 0.1 |
| (19) | (F) Water | | Balance | Balance | Balance |
| Total (% by mass) | | | 100 | 100 | 100 |
| Viscosity (mPa· s) | | | 6040 | 4250 | 4660 |
| Evaluation | | Elastomer dispersion stability (immediately after sample preparation) | A | A | A |
| | | Elastomer aggregation suppression (after rolling treatment) | E | E | E |

### [Test examples 49 to 50]

In Test examples 49 to 50, feelings in use between the non-emulsifying silicone elastomer and the emulsifying silicone elastomer were compared. The silicone elastomer of the component (A) used in Test example 50 is an emulsifying crosslinking organopolysiloxane in which the silicone chains are crosslinked by a polyglycerin chain (polyether chain) that is the hydrophilic group. On the other hand, the silicone elastomer of the component (A) used in Test example 49 is a non-emulsifying crosslinking organopolysiloxane in which the silicone chains are crosslinked by the hydrophobic group.

20 panelists rubbed the compositions of Test examples 49 and 50 on the back of their hands with their finger, respectively, and evaluated for "smoothness" and "non-stickiness" upon application by the following criteria.

### [Feeling in use (smoothness)]

A: The number of panelists that felt smoothness upon application was 10 or more;
B: The number of panelists that felt smoothness upon application was less than 10.

### [Feeling in use (non-stickiness)]

A: The number of panelists that did not feel stickiness upon application was 10 or more;
B: The number of panelists that did not feel stickiness upon application was less than 10.

In Test example 49 that used the non-emulsifying silicone elastomer, half or more of the panelists felt smoothness and did not feel stickiness. On the other hand, in Test example 50 that used the emulsifying silicone elastomer, less than half of the panelists did not feel smoothness and felt stickiness. Accordingly, in the composition of the present disclosure, it is considered that the silicone elastomer is preferably a non-emulsifying type that does not have a hydrophilic moiety in order to achieve smooth feeling in use and non-sticky feeling in use.

**[Table 11]**

| | Test Examples | | 49 | 50 |
|---|---|---|---|---|
| (1) | (A) Polysilicone-11 | | 0.84 | - |
| (2) | (A) (Dimethicone/PEG-10/15) crosspolymer^{∗}14 | | - | 0.84 |
| (3) | (B) PEG10-dimethicone ^{∗}1 | | 0.8 | 0.8 |
| (4) | (C1) Polysorbate-20^{∗}2 | | 0.8 | 0.8 |
| (5) | (C1) PEG-60 glyceryl isostearate ^{∗}3 | | 0.3 | 0.3 |
| (6) | (C2) Lauryl dimethylaminoacetic acid betaine ^{∗}12 | | 0.15 | 0.15 |
| (7) | (D) Dimethicone | | 10.5 | 10.5 |
| (8) | (D) Diphenylsiloxy phenyl trimethicone | | 1 | 1 |
| (9) | (D) Isostearic acid | | 0.23 | 0.23 |
| (10) | (E) Sodium Acrylate/Sodium Acryloyldimethyl Taurate Copolymer | | 1 | 1 |
| (11) | (E) Acrylates/C10-30 Alkyl Acrylate Crosspolymer | | 0.1 | 0.1 |
| (12) | (E) Xanthan gum | | 0.05 | 0.05 |
| (13) | (E) Succinoglycan | | 0.15 | 0.15 |
| (14) | (F) Bis-diglyceryl polyacyladipate-2 | | 1 | 1 |
| (15) | (F) Pentaerythritol tetra(2-ethylhexanoate) | | 0.5 | 0.5 |
| (16) | (F) Bis-PEG-18 methyl ether dimethyl silane | | 3 | 3 |
| (17) | (F) Glycerin | | 5 | 5 |
| (18) | (F) Dipropylene glycol | | 9 | 9 |
| (19) | (F) Polyethylene glycol | | 1 | 1 |
| (20) | (F) Sodium hyaluronate | | 0.1 | 0.1 |
| (21) | (F) Water | | Balance | Balance |
| Total (% by mass) | | | 100 | 100 |
| Viscosity (mPa· s) | | | 3900 | 3510 |
| Evaluation | | Feeling in use (smoothness) | A | B |
| | | Feeling in use (non-stickiness) | A | B |
| ^{∗}16: KSG-210, Shin-Etsu Chemical Co., Ltd. | | | | |

Formulation examples of the external composition for skin of the present disclosure are listed below. Application examples of the external composition for skin of the present disclosure are not limited to the following formulation examples.

Formulation example 1 (Table 12).

**[Table 12]**

| | Formulation example | 1 |
|---|---|---|
| (1) | (A) Crosslinking methylpolysiloxane^{∗}17 | 0.8 |
| (2) | (B) Lauryl PEG-9 polydimethylsiloxyethyl dimethicone^{∗}11 | 0.8 |
| (3) | (C1) Polysorbate-20^{∗}2 | 0.8 |
| (4) | (C1) PEG-60 glyceryl isostearate ^{∗}3 | 0.3 |
| (5) | (D) Dimethicone | 10.2 |
| (6) | (D) Diphenylsiloxy phenyl trimethicone | 1 |
| (7) | (E) Sodium Acrylate/Sodium Acryloyldimethyl Taurate Copolymer | 1 |
| (8) | (E) Acrylates/C10-30 Alkyl Acrylate Crosspolymer | 0.1 |
| (9) | (E) Xanthan gum | 0.05 |
| (10) | (E) Succinoglycan | 0.15 |
| (11) | (F) Bis-diglyceryl polyacyladipate-2 | 1 |
| (12) | (F) Pentaerythritol tetra(2-ethylhexanoate) | 0.5 |
| (13) | (F) Bis-PEG-18 methyl ether dimethyl silane | 3 |
| (14) | (F) Glycerin | 5 |
| (15) | (F) Dipropylene glycol | 9 |
| (16) | (F) Polyethylene glycol | 1 |
| (17) | (F) Sodium hyaluronate | 0.1 |
| (18) | (F) Prunus Yedoensis leaf extract | 0.1 |
| (19) | (F) Water | Balance |
| Total (% by mass) | | 100 |
| ^{∗}17: KSG-15AP, Shin-Etsu Chemical Co., Ltd. | | |

Formulation example 2 (Table 13).

**[Table 13]**

| | Formulation example | 2 |
|---|---|---|
| (1) | (A) Alkyl crosslinking polydimethylsiloxane^{∗} 18 | 0.85 |
| (2) | (B) PEG-9 polydimethylsiloxyethyl dimethicone ^{∗}10 | 0.8 |
| (3) | (C1) Polysorbate-20^{∗}2 | 0.8 |
| (4) | (C1) PEG-60 glyceryl isostearate ^{∗}3 | 0.3 |
| (5) | (C2) Lauryl dimethylaminoacetic acid betaine ^{∗} 12 | 0.15 |
| (6) | (D) Dimethicone | 10.5 |
| (7) | (D) Diphenylsiloxy phenyl trimethicone | 1 |
| (8) | (D) Isostearic scid | 0.23 |
| (9) | (D) Ionol | 0.05 |
| (10) | (E) Sodium Acrylate/Sodium Acryloyldimethyl Taurate Copolymer | 1 |
| (11) | (E) Acrylates/C10-30 Alkyl Acrylate Crosspolymer | 0.1 |
| (12) | (E) Xanthan gum | 0.05 |
| (13) | (E) Succinoglycan | 0.15 |
| (14) | (F) Bis-diglyceryl polyacyladipate-2 | 1 |
| (15) | (F) Pentaerythritol tetra(2-ethylhexanoate) | 0.5 |
| (16) | (F) Bis-PEG-18 methyl ether dimethyl silane | 3 |
| (17) | (F) Glycerin | 4 |
| (18) | (F) Dipropylene glycol | 10 |
| (19) | (F) Polyethylene glycol | 1 |
| (20) | (F) Sodium hyaluronate | 0.1 |
| (21) | (F) Trimethylsiloxysilicate | Appropriate amount |
| (22) | (F) Potassium hydroxide | Appropriate amount |
| (23) | (F) Citric acid | Appropriate amount |
| (24) | (F) Sodium citrate | Appropriate amount |
| (25) | (F) Water | Balance |
| Total (% by mass) | | 100 |
| ^{∗}18: DOW CORNING^{®} 9041 SILICONE ELASTOMER BLEND(Dow Corning Toray Co., Ltd.) | | |

The external composition for skin of the present disclosure been described based on the above-mentioned embodiments and examples.

Further problems, objects and embodiments (including modifications) of the present invention will become apparent from the entire disclosure of the present invention including the claims.

As for the numerical ranges disclosed herein, it should be understood that any numbers and ranges falling within the afore-mentioned range are specifically disclosed herein even if they are not particularly stated.

## Claims

1. An external oil-in-water emulsion composition for skin, comprising:
0.1% by mass to 8% by mass of a non-emulsifying silicone elastomer;
a polyether-modified silicone surfactant; and
a hydrophilic nonionic surfactant,
wherein the hydrophilic nonionic surfactant comprises a glycerin-derived surfactant;
the polyether-modified silicone surfactant is 0.3 part by mass or more with respect to 1 part by mass of the silicone elastomer;
the glycerin-derived surfactant is 0.2 part by mass or more with respect to 1 part by mass of the silicone elastomer;
in a case where an amphoteric surfactant is included, the amphoteric surfactant is 1.5 parts by mass or less with respect to 1 part by mass of the glycerin-derived surfactant; and
the viscosity is 9,500 mPa·s or less; wherein the viscosity can be measured by a BL viscometer (rotor No. 3, rotation speed 12 rpm) at 30°C.

2. The external oil-in-water emulsion composition for skin, according to claim 1, wherein:
the amphoteric surfactant is 0.15 part by mass to 1.5 parts by mass with respect to 1 part by mass of the glycerin-derived surfactant in a case where the amphoteric surfactant is included.

3. The external oil-in-water emulsion composition for skin, according to claim 2, wherein:
the amphoteric surfactant comprises lauryl dimethylaminoacetic acid betaine.

4. The external oil-in-water emulsion composition for skin, according to any one of claims 1 to 3, wherein:
the polyether-modified silicone surfactant has a structure in which a plurality of hydrophilic group having a polyether group is grafted from a main chain.

5. The external oil-in-water emulsion composition for skin, according to claim 4, wherein:
the main chain comprises a silicone chain.

6. The external oil-in-water emulsion composition for skin, according to any one of claims 1 to 5, wherein:
the polyether-modified silicone surfactant comprises a polyoxyethylene methylpolysiloxane copolymer.

7. The external oil-in-water emulsion composition for skin, according to any one of claims 1 to 6, wherein:
the glycerin-derived surfactant comprises a polyoxyethylene glycerin fatty acid ester.

8. The external oil-in-water emulsion composition for skin, according to any one of claims 1 to 7, wherein:
the hydrophilic nonionic surfactant further comprises a sorbitan-derived surfactant, and
the sorbitan-derived surfactant is 0.5 part by mass to 5 parts by mass with respect to 1 part by mass of the glycerin-derived surfactant.

9. The external oil-in-water emulsion composition for skin, according to any one of claims 1 to 8, further comprising:
a silicone oil.

10. The external oil-in-water emulsion composition for skin, according to any one of claims 1 to 9, wherein:
the average particle size of the silicone elastomer is 100 µm or less.

11. The external oil-in-water emulsion composition for skin, according to any one of claims 1 to 10, further comprising:
a polymer and/or a copolymer (including a crosslinked polymer) having 2-acrylamido-2-methylpropanesulphonic acid or a salt thereof as a constituent unit.

12. The external oil-in-water emulsion composition for skin, according to any one of claims 1 to 11, wherein:
the content by percentage of the silicone elastomer is 5% by mass or less with respect to the mass of the composition.

13. The external oil-in-water emulsion composition for skin, according to any one of claims 1 to 12, wherein:
the polyether-modified silicone surfactant is 0.5 part by mass or more with respect to 1 part by mass of the silicone elastomer.

14. The external oil-in-water emulsion composition for skin, according to any one of claims 1 to 13, wherein:
the silicone elastomer is a crosslinked organopolysiloxane that does not have a polyoxyalkylene group.

## Patentansprüche

1. Äußerliche Öl-in-Wasser-Emulsionszusammensetzung für die Haut, umfassend:
0,1 Massenprozent bis 8 Massenprozent eines nicht emulgierenden Silikonelastomers;
ein polyethermodifiziertes Silikon-Tensid; und
ein hydrophiles nichtionisches Tensid,
wobei das hydrophile nichtionische Tensid ein von Glycerin abgeleitetes Tensid umfasst;
das polyethermodifizierte Silikon-Tensid 0,3 Masseteile oder mehr, bezogen auf 1 Masseteil des Silikonelastomers, ausmacht;
das von Glycerin abgeleitete Tensid 0,2 Masseteile oder mehr, bezogen auf 1 Masseteil des Siliconelastomers, ausmacht;
in einem Fall, in dem ein amphoteres Tensid enthalten ist, das amphotere Tensid 1,5 Masseteile oder weniger beträgt, bezogen auf 1 Masseteil des von Glycerin abgeleiteten Tensids; und
die Viskosität 9.500 mPa-s oder weniger beträgt; wobei die Viskosität mit einem BL-Viskosimeter (Rotor Nr. 3, Rotationsgeschwindigkeit 12 U/min) bei 30°C gemessen werden kann.

2. Äußerliche Öl-in-Wasser-Emulsionszusammensetzung für die Haut nach Anspruch 1, wobei:
das amphotere Tensid 0,15 Masseteile bis 1,5 Masseteile, bezogen auf 1 Masseteil des von Glycerin abgeleiteten Tensids, beträgt, wenn das amphotere Tensid enthalten ist.

3. Äußerliche Öl-in-Wasser-Emulsionszusammensetzung für die Haut nach Anspruch 2, wobei:
das amphotere Tensid Lauryldimethylaminoessigsäurebetain umfasst.

4. Äußerliche Öl-in-Wasser-Emulsionszusammensetzung für die Haut nach einem der Ansprüche 1 bis 3, wobei:
das polyethermodifizierte Silikontensid eine Struktur aufweist, in der eine Vielzahl von hydrophilen Gruppen mit einer Polyethergruppe von einer Hauptkette aufgepfropft ist.

5. Äußerliche Öl-in-Wasser-Emulsionszusammensetzung für die Haut nach Anspruch 4, wobei:
die Hauptkette eine Silikonkette umfasst.

6. Äußerliche Öl-in-Wasser-Emulsionszusammensetzung für die Haut nach einem der Ansprüche 1 bis 5, wobei:
das polyethermodifizierte Silikon-Tensid ein Polyoxyethylen-Methylpolysiloxan-Copolymer umfasst.

7. Äußerliche Öl-in-Wasser-Emulsionszusammensetzung für die Haut nach einem der Ansprüche 1 bis 6, wobei:
das von Glycerin abgeleitete Tensid einen Polyoxyethylenglycerinfettsäureester umfasst.

8. Äußerliche Öl-in-Wasser-Emulsionszusammensetzung für die Haut nach einem der Ansprüche 1 bis 7, wobei:
das hydrophile nichtionische Tensid weiterhin ein von Sorbitan abgeleitetes Tensid umfasst, und
das von Sorbitan abgeleitete Tensid 0,5 Masseteile bis 5 Masseteile, bezogen auf 1 Masseteil des von Glycerin abgeleiteten Tensids, ausmacht.

9. Äußerliche Öl-in-Wasser-Emulsionszusammensetzung für die Haut nach einem der Ansprüche 1 bis 8, ferner umfassend:
ein Silikonöl.

10. Äußerliche Öl-in-Wasser-Emulsionszusammensetzung für die Haut nach einem der Ansprüche 1 bis 9, wobei:
die durchschnittliche Teilchengröße des Silikonelastomers 100 µm oder weniger beträgt.

11. Äußerliche Öl-in-Wasser-Emulsionszusammensetzung für die Haut nach einem der Ansprüche 1 bis 10, ferner umfassend:
ein Polymer und/oder ein Copolymer (einschließlich eines vernetzten Polymers) mit 2-Acrylamido-2-methylpropansulfonsäure oder einem Salz davon als eine konstituierende Einheit.

12. Äußerliche Öl-in-Wasser-Emulsionszusammensetzung für die Haut nach einem der Ansprüche 1 bis 11, wobei:
der prozentuale Anteil des Silikonelastomers 5 Massenprozent oder weniger, bezogen auf die Masse der Zusammensetzung, beträgt.

13. Äußerliche Öl-in-Wasser-Emulsionszusammensetzung für die Haut nach einem der Ansprüche 1 bis 12, wobei:
das polyethermodifizierte Silikon-Tensid 0,5 Masseteile oder mehr, bezogen auf 1 Masseteil des Silikonelastomers, ausmacht.

14. Äußerliche Öl-in-Wasser-Emulsionszusammensetzung für die Haut nach einem der Ansprüche 1 bis 13, wobei:
das Siliconelastomer ein vernetztes Organopolysiloxan ist, das keine Polyoxyalkylengruppe aufweist.

## Revendications

1. Composition d'émulsion huile-dans-eau externe pour la peau, comprenant :
0,1 % en masse à 8 % en masse d'un élastomère de silicone non émulsionnant ;
un agent tensioactif de silicone modifié par un polyéther ; et
un agent tensioactif non ionique hydrophile,
dans lequel l'agent tensioactif non ionique hydrophile comprend un agent tensioactif dérivé de la glycérine ;
l'agent tensioactif de silicone modifié par un polyéther représente 0,3 partie en masse ou plus par rapport à 1 partie en masse de l'élastomère de silicone ;
l'agent tensioactif dérivé de la glycérine représente 0,2 partie en masse ou plus par rapport à 1 partie en masse de l'élastomère de silicone ;
dans le cas où un agent tensioactif amphotère est inclus, l'agent tensioactif amphotère est de 1,5 partie en masse ou moins par rapport à 1 partie en masse de l'agent tensioactif dérivé de la glycérine ; et
la viscosité est de 9 500 mPa-s ou moins ; dans lequel la viscosité peut être mesurée par un viscosimètre BL (rotor n° 3, vitesse de rotation 12 tr/min) à 30°C.

2. Composition d'émulsion huile-dans-eau externe pour la peau, selon la revendication 1, dans laquelle :
le tensioactif amphotère est de 0,15 partie en masse à 1,5 partie en masse par rapport à 1 partie en masse du tensioactif dérivé de la glycérine dans un cas où le tensioactif amphotère est inclus.

3. Composition d'émulsion externe huile-dans-eau pour la peau, selon la revendication 2, dans laquelle :
le tensioactif amphotère comprend de la lauryl diméthylaminoacétique acide bétaïne.

4. Composition d'émulsion externe huile-dans-eau pour la peau, selon l'une quelconque des revendications 1 à 3, dans laquelle :
le tensioactif silicone modifié par un polyéther a une structure dans laquelle une pluralité de groupes hydrophiles ayant un groupe polyéther est greffée à partir d'une chaîne principale.

5. Composition d'émulsion externe huile-dans-eau pour la peau, selon la revendication 4, dans laquelle :
la chaîne principale comprend une chaîne silicone.

6. Composition d'émulsion externe huile-dans-eau pour la peau, selon l'une quelconque des revendications 1 à 5, dans laquelle :
le tensioactif silicone modifié par un polyéther comprend un copolymère de polyoxyéthylène méthylpolysiloxane.

7. Composition d'émulsion huile-dans-eau externe pour la peau, selon l'une quelconque des revendications 1 à 6, dans laquelle :
le tensioactif dérivé de la glycérine comprend un ester d'acide gras de polyoxyéthylène glycérine.

8. Composition d'émulsion huile-dans-eau externe pour la peau, selon l'une quelconque des revendications 1 à 7, dans laquelle :
le tensioactif non ionique hydrophile comprend en outre un tensioactif dérivé du sorbitan, et
le tensioactif dérivé du sorbitan représente 0,5 partie en masse à 5 parties en masse par rapport à 1 partie en masse du tensioactif dérivé de la glycérine.

9. Composition d'émulsion huile-dans-eau externe pour la peau, selon l'une quelconque des revendications 1 à 8, comprenant en outre :
une huile de silicone.

10. Composition d'émulsion externe huile-dans-eau pour la peau, selon l'une quelconque des revendications 1 à 9, dans laquelle :
la taille moyenne des particules de l'élastomère de silicone est inférieure ou égale à 100 µm.

11. Composition d'émulsion externe huile-dans-eau pour la peau, selon l'une quelconque des revendications 1 à 10, comprenant en outre :
un polymère et/ou un copolymère (y compris un polymère réticulé) ayant l'acide 2-acrylamido-2-méthylpropanesulfonique ou un sel de celui-ci comme unité constitutive.

12. Composition d'émulsion externe huile dans eau pour la peau, selon l'une quelconque des revendications 1 à 11, dans laquelle :
la teneur en pourcentage de l'élastomère de silicone est inférieure ou égale à 5% en masse par rapport à la masse de la composition.

13. Composition d'émulsion huile-dans-eau externe pour la peau, selon l'une quelconque des revendications 1 à 12, dans laquelle :
le tensioactif silicone modifié par un polyéther est supérieur ou égal à 0,5 partie en masse par rapport à 1 partie en masse de l'élastomère silicone.

14. Composition d'émulsion huile-dans-eau externe pour la peau, selon l'une quelconque des revendications 1 à 13, dans laquelle :
l'élastomère de silicone est un organopolysiloxane réticulé ne comportant pas de groupe polyoxyalkylène.
